# EUROPEAN PATENT APPLICATION

(11) **EP 4 840 423 A1**
(43) Date of publication of application: **18.11.2026**
(21) Application number: 26178719.6
(22) Date of filing: 13.05.2026
(51) Int. Cl.: A61F 2/38, A61F 2/30

(54) **TIBIAL PROSTHESIS AND COMPONENTS**

(30) Priority: 14.05.2025 US 202563805766 P; 23.04.2026 US 202664047332 P
(71) Applicant: Zimmer, Inc., Warsaw, IN 46580 (US)
(72) Inventor: BYRD, Brian D., North Webster, 46555 (US); YOKO, Tim, Granger, 46530 (US); REIDY, John, Warsaw, 46580 (US); MEULINK, Jacob, Warsaw, 46580 (US); VANDIEPENBOS, Jeffery A., New Paris, 46553 (US); BLAYLOCK, Jeff, Fort Wayne, 46807 (US); BARKER, Joshua, Warsaw, 46582 (US); GLASS, David L., Silver Lake, 46982 (US)
(74) Representative: Taor, Simon Edward William

(57) **Abstract**

A tibial prosthesis may include a distal surface configured for implantation in a tibia. A tibial prosthesis may include a proximal surface opposite the distal surface. A tibial prosthesis may include a periphery extending between the proximal surface and the distal surface. A tibial prosthesis may include a bore having an opening on the proximal surface and extending into a tibial keel. A tibial prosthesis may include an anterior engagement feature on the proximal surface having an asymmetric shape with a medial portion configured differently from a lateral portion. A tibial prosthesis may include a posterior engagement feature on the proximal surface, wherein the anterior engagement feature and posterior engagement feature are configured to selectively engage with either a fixed bearing or a mobile bearing.

## Description

### TECHNICAL FIELD

The present disclosure generally relates to orthopedic prostheses. More specifically, the present disclosure relates to orthopedic prosthesis systems used in knee arthroplasties, the systems having both fixed bearing and mobile bearing capability.

### BACKGROUND

Orthopedic procedures and prostheses are commonly utilized to repair or replace damaged bone and tissue in the human body. Generally, the knee is formed by the pair of condyles at the distal portion of the femur, the lower surfaces of which bear upon the correspondingly shaped proximal surface plateau of the tibia. The femur and tibia are connected by means of ligaments such as, the posterior cruciate ligament, the lateral collateral ligament, the medial collateral ligament, and the anterior cruciate ligament. These ligaments provide stability to the knee joint.

Prosthetic knee joints can be considered either constrained or unconstrained. Constrained prosthetic knee systems can include femoral and tibial prostheses, which are mechanically linked or constrained to each other to limit relative movement between the femoral and tibial prostheses. Common mechanisms for such mechanical linkage can include a hinge, band, or other linkage structure. An unconstrained prosthetic knee system includes femoral and tibial prostheses, which are not mechanically linked. An unconstrained knee utilizes the existing ligaments and other soft tissue of the patient to provide joint stability. Constrained prosthetic knees have particular applicability to cases where a patient has experienced ligament loss or the existing ligaments do not provide adequate support and stability to the knee.

Typical practice does not allow for easy conversion between an unconstrained and constrained knee systems. Rather, an extensive surgical procedure resulting in removal of both tibial and femoral orthopedic components from the bone must occur.

### OVERVIEW

This disclosure pertains generally to knee prosthesis system that includes a tibial prosthesis that can be used with both a constrained knee system and an unconstrained knee system. The tibial prosthesis can be configured for implantation on a tibia, the tibial prosthesis can include: a distal side having a tibial keel, a proximal side opposite the distal side, a periphery extending between the proximal side and the distal side, a bore having an opening on the proximal side and extending into the tibial keel, an anterior engagement feature on the proximal side and a posterior engagement feature on the proximal side; a first bearing configured to couple with the tibial prosthesis in a fixed bearing arrangement; and a second bearing configured to couple with the tibial prosthesis in a mobile bearing arrangement where the second bearing is configured to be rotatable relative the tibial prosthesis to a desired degree range, and wherein rotation of the second bearing is limited by engagement with one of the anterior engagement feature or the posterior engagement feature; wherein the second bearing is configured to receive a hinge post therein, wherein the hinge post is configured to insert in the bore, wherein the mobile bearing arrangement with the tibial prosthesis includes a first configuration where the second bearing is rotatable relative to the tibial prosthesis about a first axis approximated by a centerline of the hinge post and a second configuration where the second bearing is rotatable relative to tibial prosthesis without the hinge post received by the second bearing and the bore.

The system can be used with multiple bearings of various standard bearing configurations (sizes and shapes) as well as various femoral prostheses including various different standard shapes and sizes. In some examples, the system can be used to form a constrained knee prostheses, particularly one utilizing a hinge post. Some constrained knee prostheses with hinge posts utilize a design where the femoral prosthesis (and hinge post) are free to move generally proximal/distal relative to the tibial baseplate and the tibial bearing component.

Additionally, the inventors of the present application have recognized the need for a system that can be reconfigured for either a mobile bearing system or a fixed bearing system. The system can include components that can be assembled to form a mobile bearing constrained-knee prosthesis and a fixed unconstrained knee prosthesis.

The present system provides for additional improvements both recognized herein and those that have yet to be recognized. For example, the posterior engagement feature has a wedge-shaped or truncated wedge shaped design with one or more features such as undercut notches, projections or other coupling features along the medial and lateral sides. This configuration provides three distinct sides having locking features with the first bearing (the fixed bearing) and additional possible engagement surfaces for interaction with the second bearing (the mobile bearing). The configuration of the posterior engagement features creates enhanced retention and stability for the fixed bearing configuration as the one or more features along the medial and lateral sides work together to provide improved coupling fixation.

According to one example, the anterior engagement feature can have an asymmetric design relative to an anteroposterior axis of the tibial prosthesis. The anterior engagement feature can include a lateral portion that has a greater anterior-posterior width than a medial portion. These lateral and medial portions can be entirely separated or can be connected together such as by a small bridge or other feature placed across a window. The relative sizes (a width in the anterior-posterior direction, a length in a medial-lateral direction, etc.) of these lateral and medial portions can change relative to one another as a size of the tibial prosthesis increases. Additionally, the anterior engagement feature can include a concentric cut-out from the bore of the tibial prosthesis. This concentric cut-out medializes with size increases to the tibial prosthesis, accommodating anatomical variations due to medialization of the keel across different patient sizes. In further examples, the configuration of the anterior engagement feature introduces further improvements beyond its basic asymmetric shape. The lateral portion extends a longer length in the medial-lateral direction than the medial portion, with the relative proportions of the lateral portion and the medial portion configured to change with implant size. The window between these portions is configured to receiving a deflection arm of a locking bar (discussed below). The medial portion and the lateral portion can include engagement surfaces that act as stops to limit rotation of the second bearing according to some examples.

According to one example, the anterior engagement feature can interact with the locking bar forming a locking mechanism with the first bearing. The locking bar has a step (e.g., a tapered step) between an intermediate and a main body portion of the locking bar. This configuration creates a varying anterior-posterior thickness along the portions of the locking bar (sections such as an insertion tip and the intermediate portion have reduced thickness relative to other sections) that facilitate easier insertion of the locking bar into the knee space and into engagement with the anterior engagement feature and the first bearing. The present system is configured for medial-side-only insertion of the locking bar which improves surgical accessibility. The relief from the step provides a desired clearance during insertion.

According to one example, the first bearing can include improvements in rigidity through the addition of posterior gussets (thickened regions on the posterior adjacent and extending from a spine) and a bridge structure (e.g., a web) across the posterior of the first bearing. These features have strengthened the first bearing to reduce or prevent lift-off. Additionally, lift-off is reduced through the configuration of the posterior engagement feature including the one or more features of the posterior engagement feature discussed previously. The above features provide a more stable and durable construction of the first bearing.

According to yet another example, the tibial prosthesis can have a proximal surface design without a perimeter rail (other than a rail provided by the anterior engagement feature and the posterior engagement feature), enabling both mobile bearing rotation and compatibility with other femoral implant designs. This versatility, combined with the ability to interchange between fixed and mobile bearing configurations without removing the tibial baseplate, improves flexibility and variety in knee procedures including revision procedures.

Additionally, according to some examples, the anterior engagement feature can be placed along (at or closely adjacent such as within a few millimeters or less than a millimeter) an anterior of the periphery while the posterior engagement feature can have at least a portion be placed along (at or closely adjacent) the posterior of the periphery. This spaces the anterior engagement feature a maximum anterior-posterior distance away from the posterior engagement feature improving the tibial prosthesis's ability to reduce or prevent lift-off of the first bearing (the fixed bearing).

Prior filed United States Patent Application Publication US20240207057A1, assigned to the applicant is incorporated herein by reference in its entirety. US20240207057A1 describes a constrained knee prosthesis system with a tibial baseplate, mobile bearing, and fixed bearing that can be interchangeably used. However, the present application discloses the various features summarized above and further discussed below (in addition to other features), which provide for improvement and distinction over US20240207057A1.

### BRIEF DESCRIPTION OF THE DRAWINGS

Various embodiments are illustrated by way of example in the figures of the accompanying drawings. Such embodiments are demonstrative and not intended to be exhaustive or exclusive embodiments of the present subject matter.
FIG. 1 is a perspective view of a knee prosthesis system with a tibial prosthesis, a plurality of bearings that can be utilized with the tibial prosthesis, and a plurality of femoral prostheses for use with the tibial prosthesis and the plurality of bearings according to an example of the present application.
FIG. 2 is an exploded view illustrating various components of the knee prosthesis system configured for a constrained knee prosthesis including a selected mobile bearing and femoral prosthesis and the tibial prosthesis of FIG. 1 according to an example of the present application.
FIG. 3 is a perspective view of the system as an assembly with the tibial prosthesis, a fixed bearing, and second femoral prosthesis according to an example of the present application.
FIG. 4 is a cross-sectional view of portions of the knee prosthesis system from FIG. 3 showing the fixed bearing with the tibial prosthesis and further showing a bore plug according to an example of the present application.
FIG. 5 is a top plan view of the tibial prosthesis showing various features thereof including an anterior engagement feature and a posterior engagement feature according to an example of the present application.
FIG. 6A is a side plan view illustrating the tibial prosthesis with anterior and posterior engagement features for coupling with bearings according to an example of the present application.
FIG. 6B is a view of an anterior portion of the tibial prosthesis of FIGS. 5 and 6A illustrating the anterior engagement feature and additional components according to an example of the present application.
FIG. 6C is a cross-sectional view illustrating the anterior engagement feature of the tibial prosthesis along with additional features such as the bore of the tibial prosthesis according to an example of the present application.
FIG. 7 is a perspective view of another example of the tibial prosthesis having recesses for lobes and receiving the bore plug in the bore thereof according to an example of the present application.
FIG. 8 is a perspective view of a bushing with a tubular body and a plurality of lobes for use in a knee prosthesis system according to an example of the present application.
FIG. 9A is a top plan view of the fixed bearing of FIG. 3 with a bridge and spine according to an example of the present application.
FIG. 9B is a second plan view of the fixed bearing of FIG. 9A.
FIG. 9C is a cross-sectional view of the fixed bearing of FIGS. 9A and 9B through the bridge and gussets and other portions thereof according to an example of the present application.
FIG. 10 is a perspective view of a distal side of the fixed bearing showing anterior and posterior coupling features according to an example of the present application.
FIG. 11 is a side plan view of portions of the tibial prosthesis and the fixed bearing according to an example of the present application.
FIG. 12 is a cross-sectional view of the tibial prosthesis and the fixed bearing showing various features including an interference fit region according to an example of the present application.
FIG. 13 is a cross-sectional view illustrating showing the posterior engagement feature, the posterior coupling feature and part of the interference fit region according to an example of the present application.
FIG. 14 is a cross-sectional view of a locking bar, the fixed bearing and the tibial prosthesis according to an example of the present application.
FIG. 15 illustrates a perspective view of the knee prosthesis system showing a locking arrangement created by use of the locking bar and the anterior engagement feature with the fixed bearing according to an example of the present application.
FIG. 16 is a plan view of the locking bar including various portions and a relief feature such as a step designed for ease of insertion according to an example of the present application.
FIG. 17 is a plan view of a portion of the tibial prosthesis with the anterior engagement feature coupled to the locking bar according to an example of the present application.
FIG. 18 is a perspective view of an assembly of the mobile bearing and the tibial prosthesis according to an example of the present application.
FIG. 19 a plan view of a distal side of the mobile bearing with anterior and posterior relief cut-outs and a semi-circular projection feature according to an example of the present application.
FIG. 20 is a second plan view of an anterior side of the mobile bearing with the anterior relief cut-out and the semi-circular projection feature according to an example of the present application.
FIG. 21 is a third plan view of a posterior side of the mobile bearing with the posterior relief cut-out and arcuate feature according to an example of the present application.
FIG. 22 is a perspective view of the mobile bearing with the tibial prosthesis showing the posterior and anterior engagement features with the posterior and anterior relief cut-outs and additional features according to an example of the present application.
FIG. 23 is schematic view of the tibial prosthesis with the mobile bearing show asymmetric arrangement of the posterior engagement feature within the posterior relief cut-out which results in different distances 1800 and 1802 according to an example of the present application.
FIG. 24 is a plan view of the posterior side of parts of the knee prosthesis system including the tibial prosthesis and the mobile bearing according to an example of the present application.
FIG. 25 is a cross-sectional view illustrating the assembly of the mobile bearing with the tibial prosthesis and further showing additional features according to an example of the present application.
FIG. 26 is a table illustrating the compatibility of various stock sizes of the tibial implant with various stock sizes of the fixed bearing, various stock sizes of femoral prosthesis and various stock sizes of locking bar according to an example of the present application.
FIG. 27 is a table illustrating the compatibility of various stock sizes of the tibial implant with various stock sizes of the mobile bearing, various stock sizes of femoral prosthesis and various stock sizes of locking bar according to an example of the present application.
FIG. 28 is a plan top view of another example of a tibial prosthesis having another example of a posterior engagement feature according to an example of the present application.
FIG. 29 is a plan side view of the tibial prosthesis of FIG. 28 showing the posterior engagement feature has an angulated wedge configuration in the anterior-posterior direction according to an example of the present application.
FIGS. 30-32 shows another example of a tibial prosthesis having yet another example of a posterior engagement feature according to an example of the present application.
FIG. 33 shows the tibial prosthesis of FIGS. 30-32 includes a ramp at a proximal side thereof and a groove within the bore thereof.
FIG. 34 shows the tibial prosthesis of FIG. 33 with the ramp used to facilitate insertion of a tool to access a snap ring, a bushing and/or a bore plug according to an example of the present application.
FIG. 35 is a perspective view of a mobile bearing similar to that of FIG. 22 but with a different construction, FIG. 35 shows the mobile bearing with the tibial prosthesis of FIGS. 30-34 showing the posterior and anterior engagement features with the posterior and anterior relief cut-outs and additional features according to an example of the present application.
FIG. 36 is a perspective view of a distal side of a fixed bearing having a construction similar to that of FIG. 10 but modified for use with the tibial prosthesis of FIGS. 30-35, FIG. 36 showing anterior and posterior coupling features of the fixed bearing according to an example of the present application.
FIG. 37 is a plan view of the locking bar according to an example of the present application.
FIG. 38 is a plan view of another example of a tibial prosthesis having an uninterrupted distal keel according to an example of the present application.

### DETAILED DESCRIPTION

The present application relates to a system that utilizes a single tibial prosthesis that can be used for both unconstrained and constrained knee prosthesis systems. The system disclosed herein can include a set of components, e.g., a single tibial prosthesis (sometimes called a baseplate or tray herein), a plurality of femoral prostheses, etc. The set of components can be configured to operate with one of a plurality of tibial bearing components (simply referred to as a bearing or bearings herein). In examples, the system can include a mobile bearing and a fixed bearing, which can be substituted at surgeon discretion. The tibial prosthesis can be configured to interact with the mobile bearing to limit rotation (or other movement) of the bearing. Such limiting of movement can be limiting of rotation about an axis defined by a hinge post of a constrained knee prosthesis system according to some examples. However, according to other examples the mobile bearing can be used without the hinge post in an unconstrained knee system. Limiting of movement of the mobile bearing can be accomplished by the mobile bearing rotating and engaging with one or more features (e.g., one of a posterior engagement feature or an anterior engagement feature) of the tibial prosthesis. In another example, a distinct fixed bearing such as for another unconstrained knee system is selected for use rather than the mobile bearing. In this example, the fixed baring is configured to couple with the posterior engagement feature, the anterior engagement feature and additionally with a locking bar (e.g., a clip, insert or the like) to prevent movement (e.g., rotation of the fixed bearing relative to the tibial prosthesis) and reduce or prevent micro-motion of the fixed bearing.

As used herein, "micro-motion" refers to the small motions that may exist between prosthesis components, such as between the tibial baseplate and the tibial bearing component, respectively, upon application of force. Such small motions may occur as a result of material deformation in one or both of the interacting components, or may result from slight spaces or clearances therebetween, for example. Micro-motion is distinguished from "mobile bearing arrangement" (also referred to as application, configuration, assembly or the like) which experience relatively larger motions as the tibial bearing component articulates with respect to the tibial prosthesis (such as by sliding or rotating) along a desired motion path.

As used herein, a "fixed bearing arrangement" is a prosthesis assembly in which the tibial bearing component is seated atop the tibial prosthesis in a final, locked, and secured position. In this secured position, lift-off of the tibial bearing component from the tibial prosthesis as well as large movement of the tibial bearing component relative to the tibial baseplate such as rotation is prevented during natural articulation of the knee. Some micro-motion may exist between the tibial bearing component and tibial prosthesis in a fixed bearing arrangement.

As used herein, the terms "proximal" and "distal" should be given their generally understood anatomical interpretation. The term "proximal" refers to a direction generally toward the torso of a patient, and "distal" refers to the opposite direction of proximal, i.e., away from the torso of a patient. It should be understood that the use of the terms "proximal" and "distal" should be interpreted as though the patient were standing with the knee joint in extension despite the apparatuses described herein generally being used with the knee joint in flexion. The intent is to differentiate the terms "proximal" and "distal" from the terms "anterior" and "posterior". As used herein, the terms "anterior" and "posterior" should be given their generally understood anatomical interpretation. Thus, "posterior" refers to a rear of the patient, e.g., a back of the knee. Similarly, "anterior" refers to a front of the patient, e.g., a front of the knee. Thus, "posterior" refers to the opposite direction of "anterior", Similarly, the term "lateral" refers to the opposite direction of "medial".

As used herein, the "periphery" of a tibial prosthesis refers to any periphery as viewed in a top plan view, e.g., in a generally transverse anatomical plane. Alternatively, the periphery of a tibial prosthesis may be any periphery as viewed in the bottom plan view, e.g., in a generally transverse plane and looking at the distal surface adapted to contact a resected proximal surface of a tibial bone. The periphery includes the thickness of the tibial prostheses that extends between a proximal side and a distal side of the tibial prosthesis.

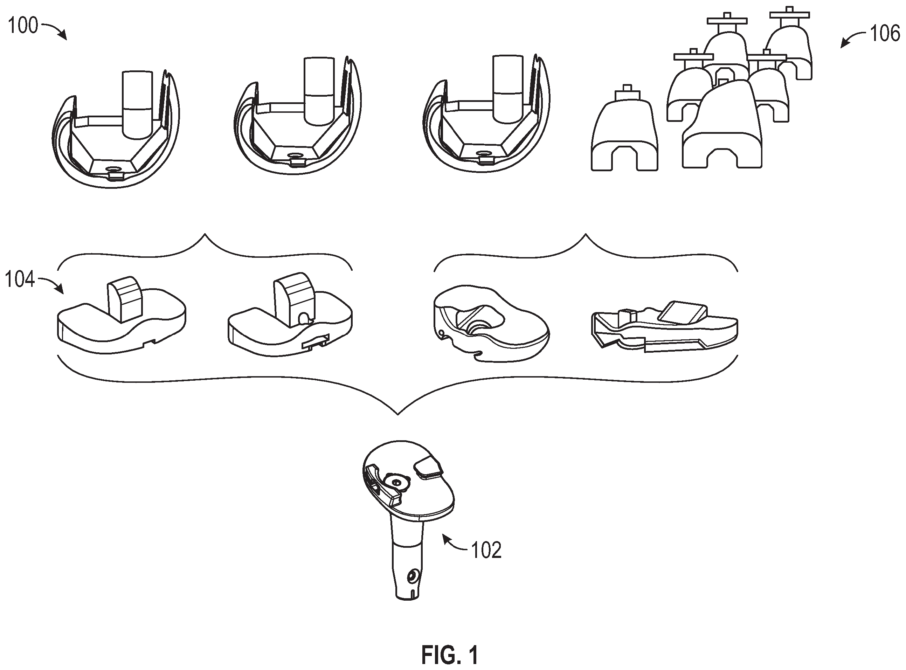

FIG. 1 illustrates a knee prosthesis system 100 (simply called a system 100 herein). The system 100 can include a tibial prosthesis 102, a plurality of bearings 104 and a plurality of femoral prostheses 106. Additional components of the system 100 can be used but are not illustrated in FIG. 1 for simplicity.

The tibial prosthesis 102 can comprise a single component that can be implanted in the tibia, and then is not disturbed (i.e., not explanted) when the plurality of bearings 104 and the plurality of femoral prostheses are selected and/or swapped/substituted as desired. The construction of the tibial prosthesis 102 will be further illustrated and discussed subsequently.

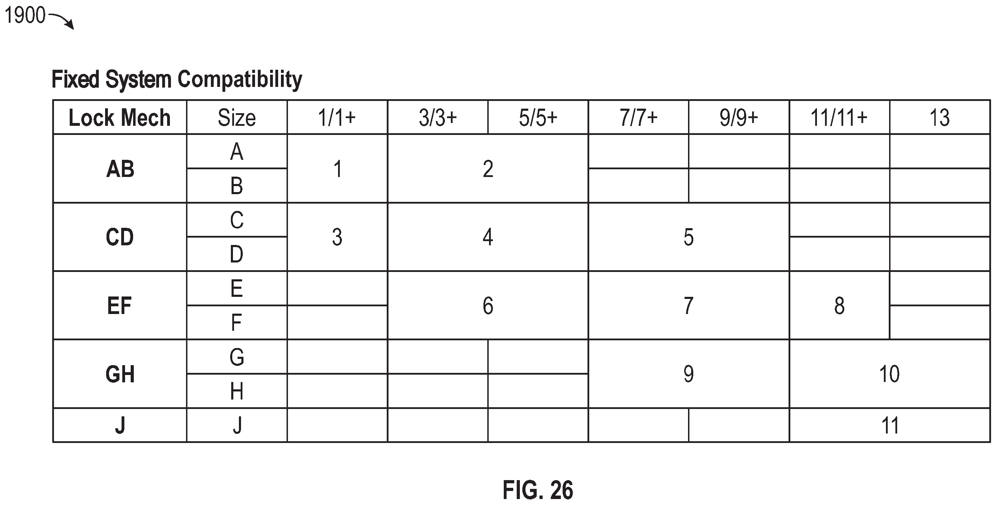

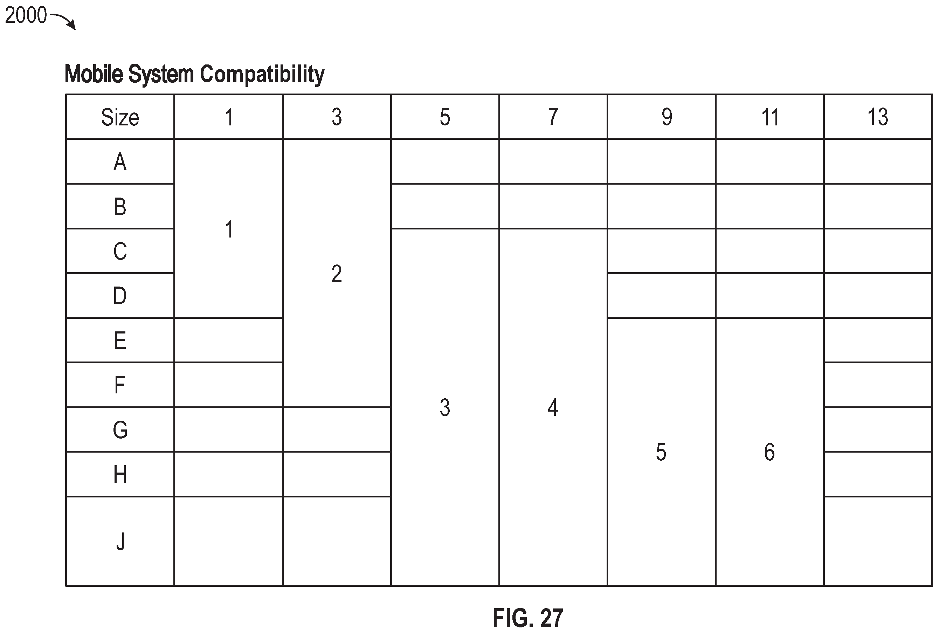

The plurality of bearings 104 can include those configured for fixed bearing arrangement and those configured for mobile bearing arrangement. Additional sub-categories of the fixed and mobile bearing arrangements are also contemplated as desired and can include those used for constrained knee, unconstrained knee, limb salvage, etc. One or more (including all) of the plurality of bearings 104 can have different standard stock sizes and shapes such that standard bearing families are utilized. For simplicity, the various different sizes of the plurality of bearings 104 are not illustrated in FIG. 1 but examples of different stock sizes are further described in reference to FIGS. 26 and 27. Additionally, although the present application focuses on only two exemplary standard bearing families, it is understood (and also illustrated in FIG. 1) that additional standard bearing families (e.g., four or more) are contemplated for use with the tibial prosthesis 102 as part of the system 100. The plurality of bearings 104 are configured to couple with the tibial prosthesis 102 as further discussed and illustrated herein.

The plurality of femoral prostheses 106 can include those configured for use in the fixed bearing arrangement and those configured for use in the mobile bearing arrangement. Additionally the plurality of femoral prostheses 106 can include those specifically configured for additional categories of the fixed and mobile bearing arrangements including those used for constrained knee, unconstrained knee, limb salvage, etc. One or more (including all) of the plurality of femoral prostheses 106 can have different standard sizes and shapes such that standard femoral prosthesis families are utilized. For simplicity, the various different sizes of the plurality of femoral prostheses 106 are not illustrated in FIG. 1 but examples are further described in reference to FIGS. 26 and 27. Although the present application illustrates four different standard prostheses families, it is contemplated additional families can be used with the tibial prosthesis 102 as part of the system 100. Respective of the plurality of femoral prostheses 106 are configured for articulation with respective of the plurality of bearings 104.

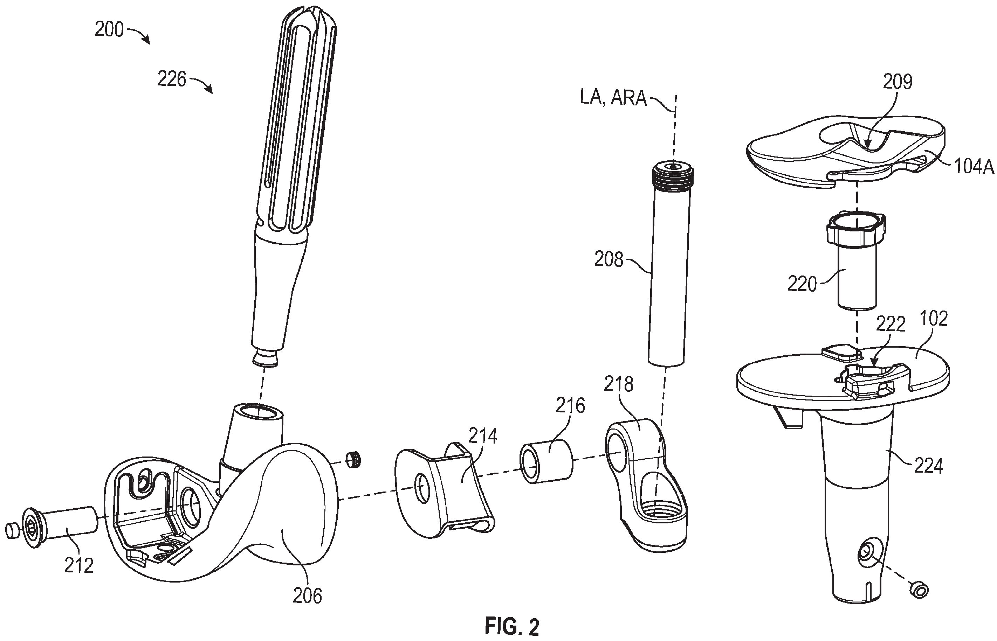

FIG. 2 shows the system 100 with various components selected for a constrained knee configuration. The system 100 can include the tibial baseplate 102 as previously discussed and illustrated in FIG. 1, a tibial bearing component (one of the plurality of tibial bearing components 104 of FIG. 1) that is specifically configured as a mobile bearing 104A (sometime called a meniscal component, poly, articular component or bearing) and a femoral prosthesis 106A (one of the plurality of femoral prostheses 106 of FIG. 1) configured for use with the mobile bearing 104A. The femoral prosthesis 106A can include various components including a hinge post 208, hinge axle 212, poly box 214, axle bushing 216, shackle 218 and a bushing 220.

The tibial bearing component 104A can be configured to coupled to and can be positioned atop a proximal surface of the tibial prosthesis 102. The tibial bearing component 104A (and those described subsequently whether fixed or mobile) can be formed of polymer material such as Ultra-High-Molecular-Weight-Polyethylene ("UHMWPE"), or the like. The tibial bearing component 104A can be configured for articulation with the femoral prosthesis 106A through knee joint flexion and extension as known in the art. The system 100 has the femoral prosthesis 106A and the tibial baseplate 102 mechanically linked to one another. This is accomplished by the hinge post 208 and other components and features further illustrated and discussed in FIG. 2 and others of the FIGURES. The hinge post 208 is coupled to the femoral prosthesis 106A and is received within a recess 209 of the tibial bearing component 104A and a bore 222 of the tibial baseplate 102.

FIG. 2 shows an exploded view of the system 100 according to one exemplary configuration. The hinge post 208 is configured to connect to femoral prosthesis 106A via the shackle 218, the axle bushing 216, and the hinge axle 212. A distal portion of the shackle 218 is received in the recess 209 in the tibial bearing component 104A and the distal portion is threaded or otherwise connected to the hinge post 208. The hinge post 208 extends distally through the recess 209 of the tibial bearing component 104A and is received in the bore 222 of the tibial prosthesis 102. The bore 222 of the tibial prosthesis 102 that receives the hinge post 208 can at least partially be formed by a stem 224 (sometimes called a stem, stem assembly, stem and keel assembly or the like) of the tibial prosthesis 102. The stem 224 is a distally extending retention feature(s) configured for insertion into a prepared recess of the tibia. The hinge post 208 can be moveable, e.g., rotatable or capable of distraction, relative to the tibial bearing component 104A and/or the tibial prosthesis 102. The hinge post 208 can be rotatably connected to femoral prosthesis 106A via the hinge axle 212. Thus, a longitudinal axis LA that defines a centerline of the hinge post 208 can define an axis of rotation/articulation ARA for the knee joint as the femoral prosthesis 106A and the tibial prosthesis 102 are mechanically linked.

When assembled, the shackle 218 can be placed between opposing walls of poly box 214. When assembled on the hinge axle 212, the axle bushing 216 additionally resides within an aperture on a proximal portion of the shackle 218. The shackle 218 and hinge post 208 can be formed from suitable materials such as a titanium alloy, a cobalt-chromium alloy, or the like, while the axle bushing 216 and the poly box 214 can be formed from different materials such as plastic, e.g., UHMWPE. The axle bushing 216 can act as a bearing between the shackle 218 and the hinge axle 212. The poly box 214 can act as a bearing between the femoral prosthesis 106A and the shackle 218.

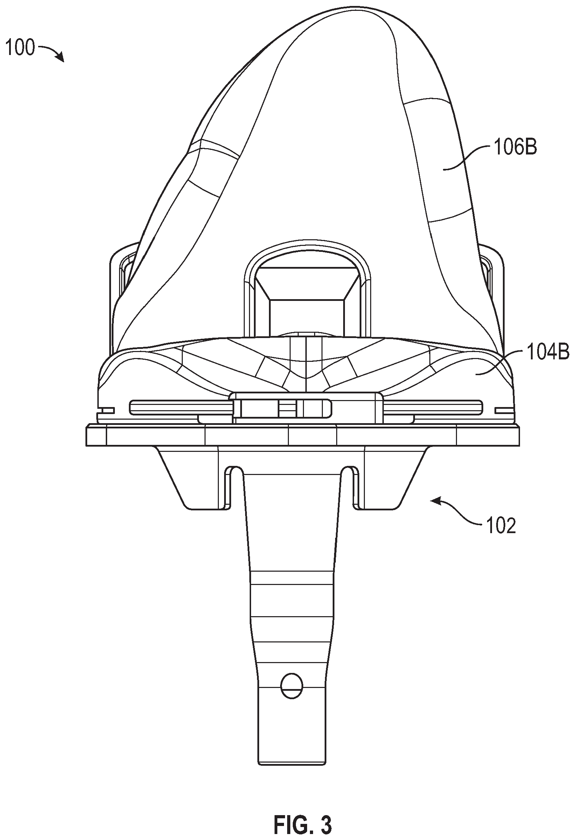

The assembly of FIG. 2 shows the system 100 configured using components where luxation of the knee is not limited by a capture element or other feature of the system 100. However, configurations limiting luxation are contemplated for use herein. Examples of such configurations are discussed in United States Patent Application Publications US20240207063A1, US20240207055A1 and US 20240207058A1, the entire disclosures of each of which are incorporated herein in their entirety. Thus, the system 100 provides components that, when assembled, are configured for full luxation. Thus, soft tissue of the knee is relied upon to limit distraction between the femoral prosthesis 106A and the tibial prosthesis 102 and the tibial bearing component 104A. The bushing 220 can be configured to insert into the bore 222 of at least the tibial prosthesis 102. In some examples, the bushing 220 can also insert into or through the recess 209 (FIG. 3) of the tibial bearing component 104A in some examples. The bushing 220 can be configured to receive at least a portion of the hinge post 208. The bushing 220 can act as a bearing between the hinge post 208 and the tibial prosthesis 102. The hinge post 208 can be moveable generally proximal/distal (as well as rotationally in some examples) relative to the bushing 220. Thus, according to some examples the hinge post 208 is configured for luxation and rotation relative to the tibial prosthesis 102.

FIG. 3 is a plan view of the system 100 configured as an unconstrained knee assembly using the tibial prosthesis 102, a tibial bearing component (one of the plurality of tibial bearing components 104 of FIG. 1) that is specifically configured as a fixed bearing 104B (again sometime called a meniscal component, poly, articular component or bearing) and a femoral prosthesis 106B (one of the plurality of femoral prostheses 106 of FIG. 1) configured for use with the fixed bearing 104B. The fixed bearing 104B is positioned atop the tibial prosthesis 102, which is configured to be implanted on a tibia.

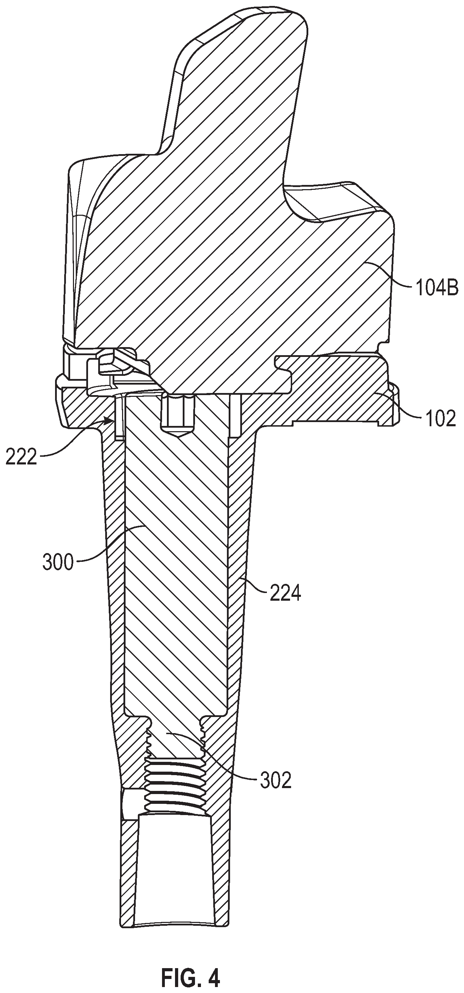

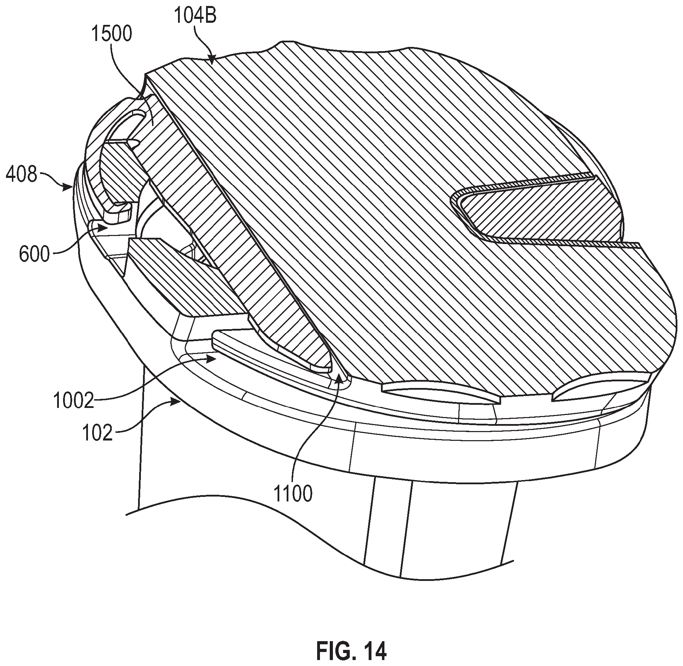

FIG. 4 is a cross-sectional view of some of the components the system 100 of FIG. 3, specifically, the fixed bearing 104B coupled with the tibial prosthesis 102. It should be noted in FIGS. 3 and 4, an additional component, a locking bar is not specifically shown but would be utilized in the configuration of FIGS. 3 and 4 for fixed bearing arrangement. The locking bar is shown and discussed in reference to FIGS. 14-17.

FIG. 4 additionally shows the bore 222, which extends into and is at least partially formed by the stem 224. The bore 222 is configured to receive a bore plug 300 therein as illustrated in FIG. 4. The bore plug 300 can be formed a polymer material (e.g., UHMWPE). As shown in FIG. 4, the bore plug 300 can include a distal coupling feature 302 configured to couple with a mating feature of the tibial prosthesis 102. This distal coupling feature 302 can be a thread as shown, however additional examples contemplate other suitable mechanical features or linkages. For example, the bore plug 300 can be alternatively retained by a diametric press fit rather than a thread, according to other examples.

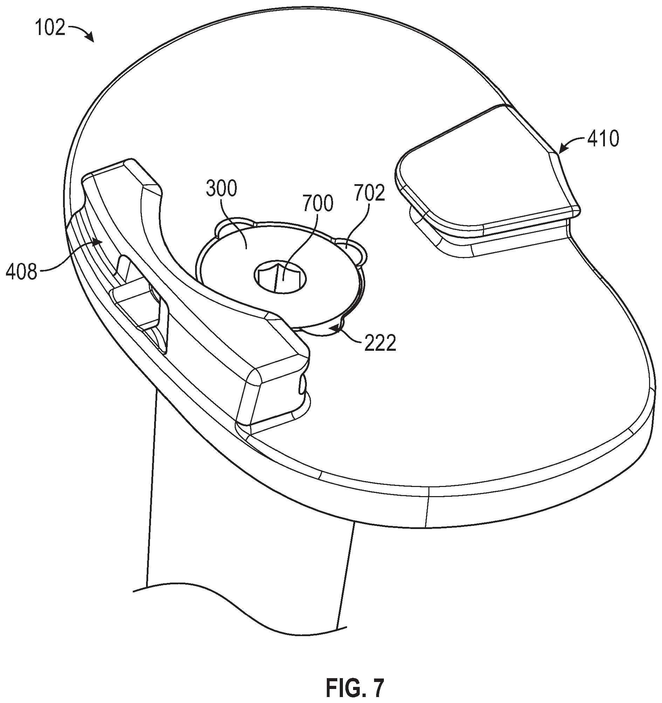

Although shown with respect to the fixed bearing configuration of FIGS. 3 and 4, the bore plug 300 can be used in mobile bearing arrangements according to further examples. The bore plug 300 is additionally shown in FIG. 7 from a proximal side. The bore plug 300 can be generally flush with or recessed distal of a proximal surface of the tibial prosthesis so as not to impede coupling or movement with the bearing with the tibial prosthesis. The bore plug 300 can include a driving and insertion feature 700 at a proximal end thereof and is insertable via this insertion feature 700 into the bore 222 via an opening 702 to the bore 222 at the proximal side. The bore 222 is designed to receive the bore plug 300, which is shown inserted within the bore 222. The bore plug 300 serves to fill substantially an entirety of the bore 222 when the tibial prosthesis is used with the fixed bearing 104A (or in some cases a mobile bearing), preventing debris from entering the bore 222. Additionally, the bore plug 300 can be removed from the bore 222 via the opening on the proximal side using the driving and insertion feature 700, for example.

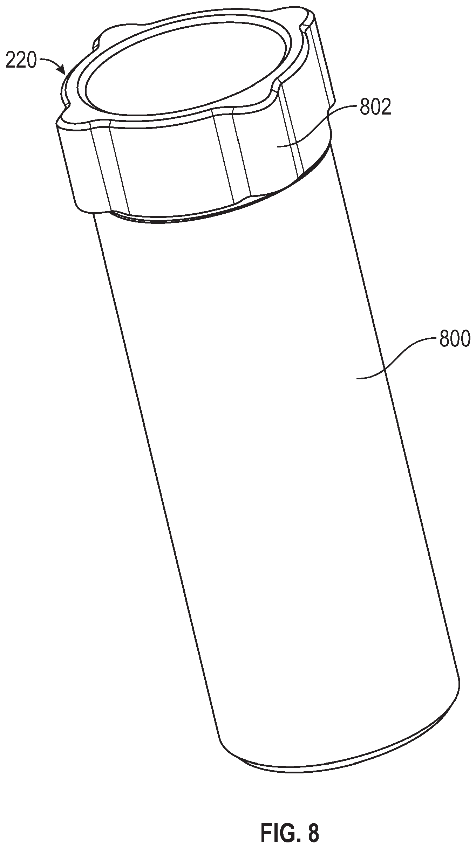

Referring now to FIG. 8, the bushing 220 also shown previously in FIG. 2 is shown. The bushing 220 includes a tubular elongate main body 800 configured for insertion into the bore 222 (FIGS. 2 and 4-7). The bushing 220 can include a plurality of lobes 804 at a proximal end thereof. These plurality of lobes 804 can be anti-rotation features that are configured to be received in sized and shaped recess features of the bore 222 (FIG. 7). The plurality of lobes 804 are configured to interact with the recess features of the bore 222 to counteract rotation of the bearing 220 when inserted into the bore 222 (FIG. 7).

The bore 222 is configured to selectively receive either the bore plug 300 or the tibial bushing 220 (FIG. 2). Thus, the tibial prosthesis 102 is configured to selectively receive either the bore plug 300 (FIGS. 4 and 7) or the tibial bushing (FIGS. 2 and 8) in the bore 222. The bore plug 300 can be inserted into bore 222 from a proximal location when using either a fixed bearing or mobile bearing configuration. Both the bore plug 300 and tibial bushing 220 are configured to be selectively inserted into and removed from bore 222 while maintaining the tibial baseplate implanted in a tibia, enabling conversion between mobile bearing and fixed bearing configurations.

Referring to FIGS. 2, 4, 7 and 8, the present application includes a method of converting a knee prosthesis between configurations involves removing bore plug 300 from bore 222 in a tibial baseplate while maintaining the tibial baseplate implanted in a tibia, inserting a tibial bushing into the bore 222, and coupling a mobile bearing with a hinge post to enable rotation about a centerline of the hinge post. Prior to inserting the tibial bushing, a fixed bearing can be decoupled from the tibial baseplate while maintaining the tibial baseplate implanted in the tibia.

The knee prosthesis can be converted by removing a tibial bushing from bore 222 in a tibial baseplate from a proximal location while maintaining the tibial baseplate implanted in a tibia, inserting the bore plug 300 into bore 222, and coupling a fixed bearing to the tibial baseplate. The removing the tibial bushing from bore 222 enables conversion from a mobile bearing configuration to a fixed bearing configuration. Prior to removing the tibial bushing, a mobile bearing and hinge post can be decoupled from the tibial baseplate while maintaining the tibial baseplate implanted in the tibia.

When preparing a knee prosthesis for implantation, a selection can be made between a mobile bearing configuration and a fixed bearing configuration. For the mobile bearing configuration, a tibial bushing can be inserted into bore 222. For the fixed bearing configuration, bore plug 300 is inserted into bore 222. The tibial bushing is configured to receive a hinge post that couples with a mobile bearing to enable rotation about a centerline of the hinge post. Both bore plug 300 and the tibial bushing are configured to be selectively inserted into and removed from bore 222 while maintaining the tibial baseplate implanted in a tibia to enable conversion between the mobile bearing configuration and fixed bearing configuration.

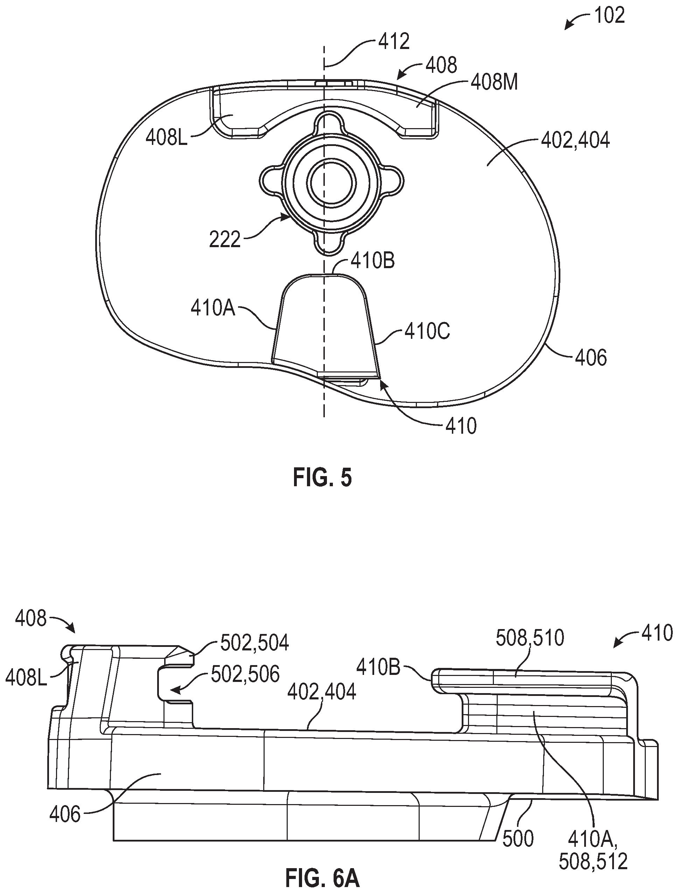

FIG. 5 is a plan view of a proximal side of the tibial prosthesis 102 shown in isolation from other components. The tibial prosthesis 102 can be configured as a baseplate or tray and can include a proximal side 402 with a proximal surface 404, a distal side (not shown in FIG. 5 but shown subsequently), a periphery 406, an anterior engagement feature 408 and a posterior engagement feature 410. FIG. 5 additionally shows the bore 222, which is medialized so as to be offset relative to an anteroposterior axis 412 that divides a medial side of the tibial prosthesis 102 from a lateral side of the tibial prosthesis. The anterior engagement feature 408 is asymmetrically shaped with respect to the anteroposterior axis 412 and includes a lateral portion 408L and a medial portion 408M. The lateral portion 408L is differently configured from the medial portion 408M having a different size, shape, and relative location relative to the anteroposterior axis 412. As shown in FIG. 5, the lateral portion 408L has a greater anterior-posterior width (greater anterior-posterior distance/extent) and has a greater medial-lateral length (extent/distance) than the medial portion 408M. Thus, the medial portion 408M is relatively smaller in size than the lateral portion 408L including in at least two dimensions.

The tibial baseplate 102 can be configured to be inserted into the tibia. The proximal side 402 with the proximal surface 404 can oppose the distal side (shown in FIG. 6A). The proximal surface 404 can be substantially flat or can be angulated to a desired degree such as proximal-distal and anterior-posterior in a manner known in the art. The proximal surface 404 can be generally opposite the distal surface, and the periphery 406 of the tibial baseplate 102 can extend between the distal side and the proximal side 402. The bore 222 can have the opening at the proximal surface 404 of the proximal side.

The anterior engagement feature 408 can be a projection from the proximal surface 404 of the tibial prosthesis 102, designed to interact with the mobile bearing 104A to limit rotation or receive the locking bar in the fixed bearing configuration as further shown and described. According to some examples, the anterior engagement feature 408 can be a recess below the proximal surface 404 rather than being a projection. The anterior engagement feature 408 can be positioned at or closely adjacent (within a few millimeters or less than a millimeter) the periphery 406 on an opposing side from the posterior engagement feature 410.

The posterior engagement feature 410 can be a second projection from the proximal surface 404 of the tibial prosthesis 102. This posterior engagement feature 410 has a wedge or truncated wedge shape with coupling features arranged on at least a generally anterior facing side 410B (first side), a lateral-posterior extending side 410A (second side), and a medial-posterior extending side 410C (third side). At least a portion of the posterior engagement feature 410 can extend to the periphery 406 of the tibial prosthesis 102 such as on the lateral side thereof shown in FIG. 5 relative to the anteroposterior axis 412. Although shown as a second projection in FIG. 5, the posterior engagement feature 410 can be a recess below the proximal surface 404 rather than being a projection.

According to the example of FIG. 5, the anterior engagement feature 408 and the posterior engagement feature 410 can be the only projections from the proximal surface 404 thereby allowing for the rotation of the mobile bearing 104A (FIG. 2).

FIG. 6A shows the tibial prosthesis of FIG. 5 from a lateral side in plan view. As shown in FIG. 6A the tibial prosthesis includes the proximal side 402, the proximal surface 404, a distal side 500, the periphery 406, the anterior engagement feature 408 including the lateral portion 408L, the posterior engagement feature 410 with the generally anterior facing side 410B and the lateral-posterior extending side 410A. The tibial prosthesis 102 additionally includes one or more coupling features 502 for the anterior engagement feature 408 such as a projection 504 and a recess or groove 506. The tibial prosthesis 102 additionally includes one or more coupling features 502 along the anterior facing side 410B and the lateral-posterior extending side 410A of the posterior engagement feature 410, the one or more coupling features 508 can include a projection 510 and a recess or groove 512.

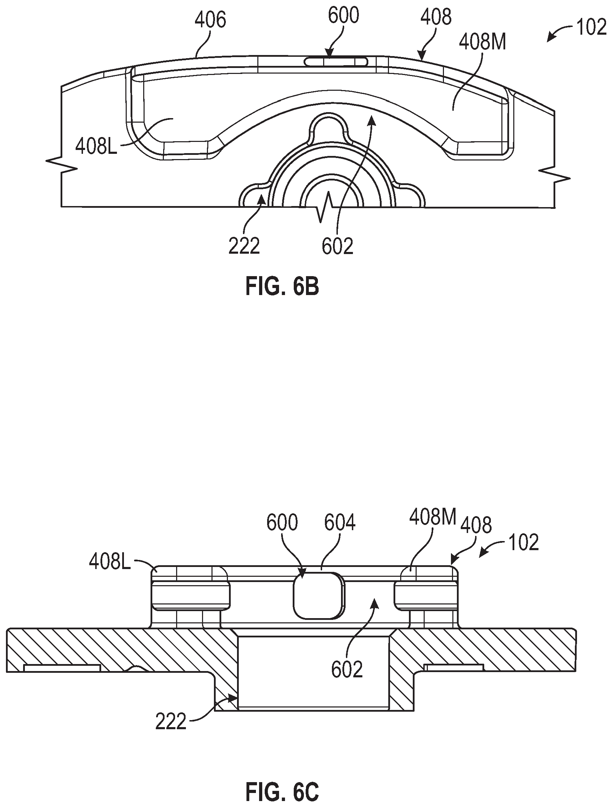

FIG. 6B shows an anterior portion of the tibial prosthesis of FIG. 5 showing a portion of the bore 222, the periphery 406 and the anterior engagement feature 408 including the lateral portion 408L and the medial portion 408M. The anterior engagement feature 408 additionally includes a window 600 configured to receive an end of a deflection arm of the locking bar. The window 600 can in some cases entirely separate the medial portion 408M from the lateral portion 408L. However, in other cases a small bridge or strip of material can remain connecting the medial portion 408M with the lateral portion 408L.

The anterior engagement feature 408 can additionally include a concentric cut-out 602 (a notch and/or groove) around and spaced from at least a portion of the bore 222. The concentric cut-out 602 medializes in position concomitant with a medial shift of position of the bore 222 relative to the anteroposterior axis 412 (FIG. 5) when the tibial prosthesis moves from a first standard size to a second larger standard size.

FIG. 6C is a cross-sectional view of the tibial prosthesis 102 through the bore 222 showing the anterior portion thereof including the anterior engagement feature 408 including the lateral portion 408L and the medial portion 408M. FIG. 6C additionally shows the window 600 and the concentric cut-out 602 that is relief from the bore 222. FIG. 6C also shows a platform 604 that is proximal of the window 600 and connects the lateral portion 408L with the medial portion 408M.

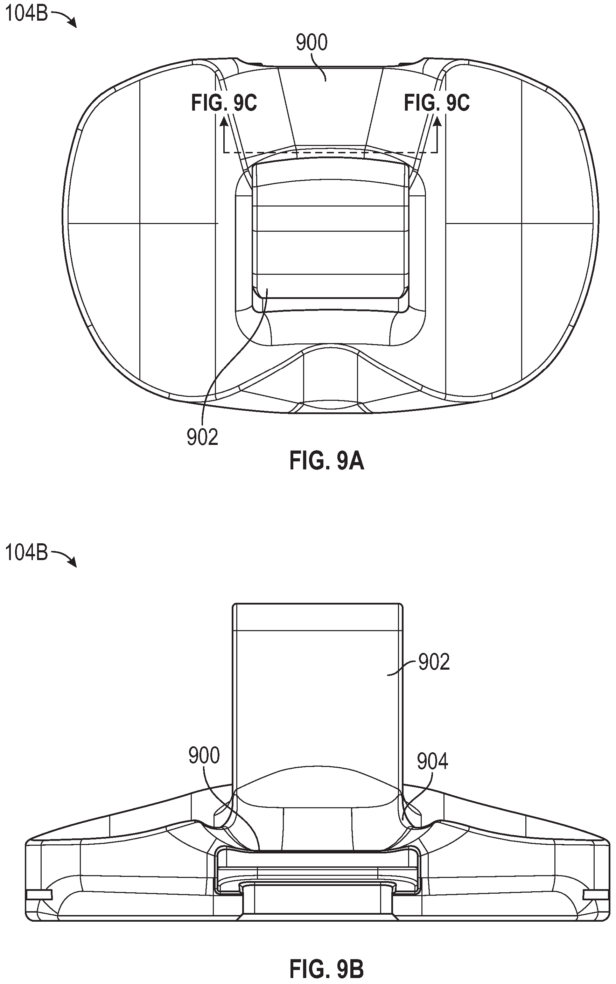

FIGS. 9A and 9B show the fixed bearing 104B in further detail. The fixed bearing 104B can include a medial compartment and a lateral compartment (not numbered) configured as dish shaped portions to articulate with respective condyles of the femoral prothesis as known in the art. The fixed bearing 104B can include a bridge 900 and a spine 902. The spine 902 can be located between the medial compartment and the lateral compartment. The bridge 900 can be posterior of the spine 902 and can extend between the lateral compartment and the medial compartment. The bridge 900 can extend from the spine 900 to at or adjacent a posterior periphery of the fixed bearing 104B. The bridge 900 can be a thin strip of material (e.g., a few millimeters or less than a millimeter in thickness) connecting the medial compartment with the lateral compartment posterior of the spine 902.

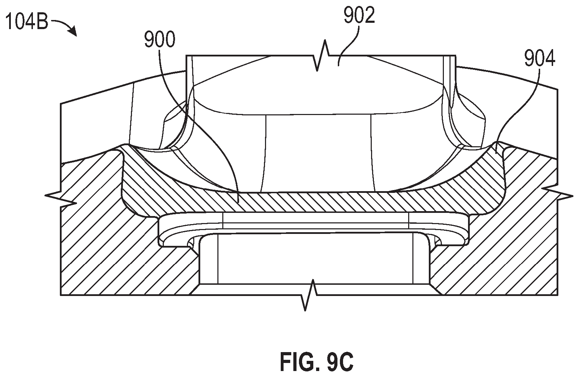

FIG. 9C is a cross-section of the fixed bearing 104B posterior of the spine 902. The cross-section of FIG. 9C is through the bridge 900 and additionally through gussets 904, which provide additionally material at connection between the bridge 900 with the medial compartment and the lateral compartment. The gussets 904 can extend up to and can form part of the posterior of the spine 902 and can additionally extend rearward toward and/or to the posterior periphery of the fixed bearing 104B. The gussets 904 and the bridge 900 are configured to increase rigidity and reduce liftoff of the fixed bearing 104B from the tibial prosthesis.

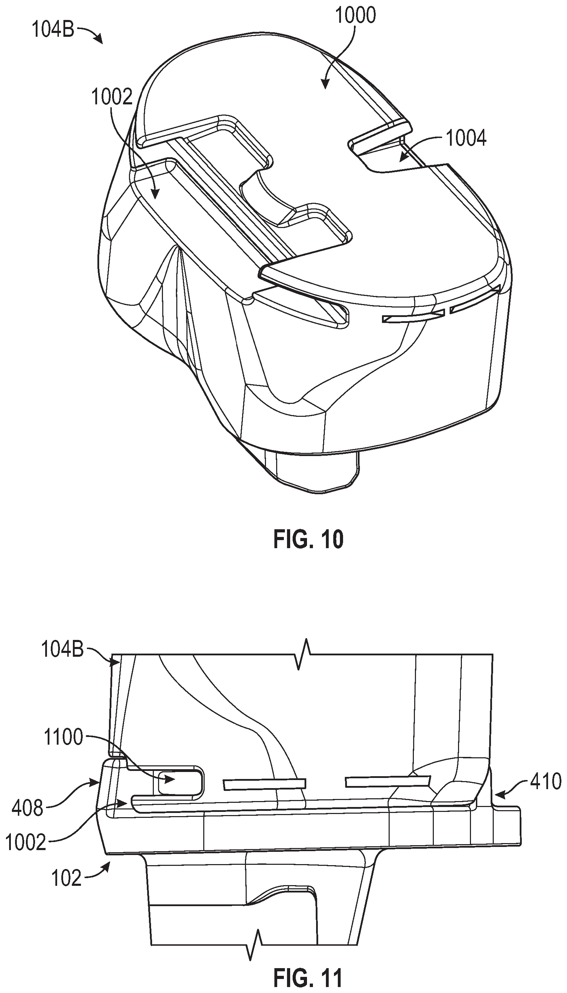

FIG. 10 illustrates a distal side 1000 of the fixed bearing 104B. The fixed bearing 104B can include one or more coupling features including an anterior coupling feature 1002 and a posterior coupling feature 1004. The one or more coupling features including the anterior coupling feature 1002 and the posterior coupling feature 1004 can be recesses (e.g., notches, grooves, etc.) and/or projections or other features configured to couple with or otherwise interact with the anterior engagement feature and the posterior engagement feature, respectively.

FIG. 11 shows the fixed bearing 104B seated down on the tibial prosthesis 102. As shown the anterior coupling feature 1002 receives the anterior engagement feature 408 and the posterior coupling feature (not numbered) receives the posterior engagement feature 410. The anterior coupling feature 1002 and the anterior engagement feature 408 together form an asymmetrically shaped passage 1100 that receives the locking bar (shown in FIGS. 14-17).

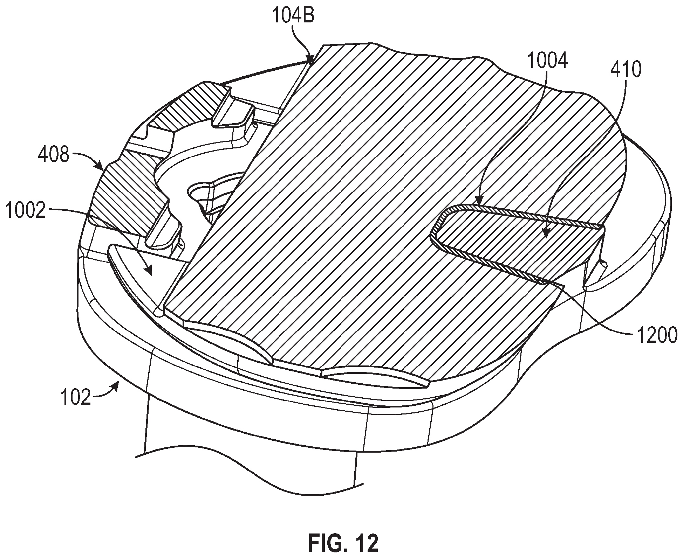

FIG. 12 is perspective view showing a cross-section of the fixed bearing 104B and a cross-section of the tibial prosthesis 102. The cross-section of FIG. 12 extends through the anterior coupling feature 1002, the anterior engagement feature 408, the posterior coupling feature 1004 and the posterior engagement feature 410. FIG. 12 additionally shows an interference fit region 1200 between the posterior coupling feature 1004 and the posterior engagement feature 410.

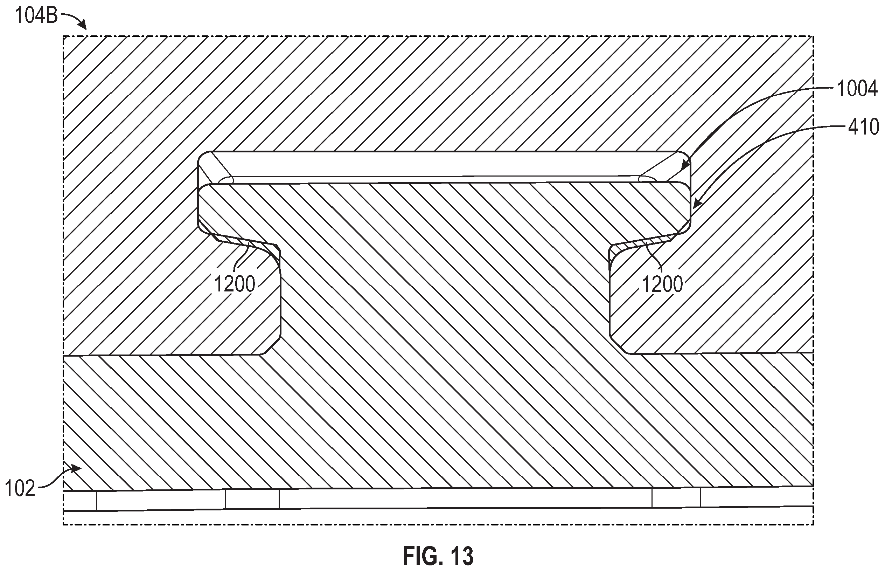

FIG. 13 is a cross-sectional view of portions of the fixed bearing 104B and the tibial prosthesis 102. The cross-section of FIG. 13 extends through the posterior coupling feature 1004 and the posterior engagement feature 410 and the interference fit region 1200 therebetween formed by mating of projections and grooves of the posterior coupling feature 1004 and the posterior engagement feature 410.

FIG. 14 is a perspective view showing a cross-section of the fixed bearing 104B and cross-section of the tibial prosthesis 102 similar to that of FIG. 12 but additionally illustrating a cross-section of a locking bar 1500 received within the asymmetrical shaped passage 1100. As shown in FIG. 14, a portion of the locking bar 1500 such as an end part of a deflection arm is received by the window 600 when the locking bar 1500 is fully inserted and captured between/by the anterior coupling feature 1002 and the anterior engagement feature 408. As shown in FIG. 14, the locking bar 1500 has a change of width in an anterior-posterior direction along an elongate length when inserted into the asymmetrical shaped passage 1100. This change in width generally allows for ease of insertion of the locking bar 1500 from a medial side of the fixed bearing 104B and the tibial prosthesis 102.

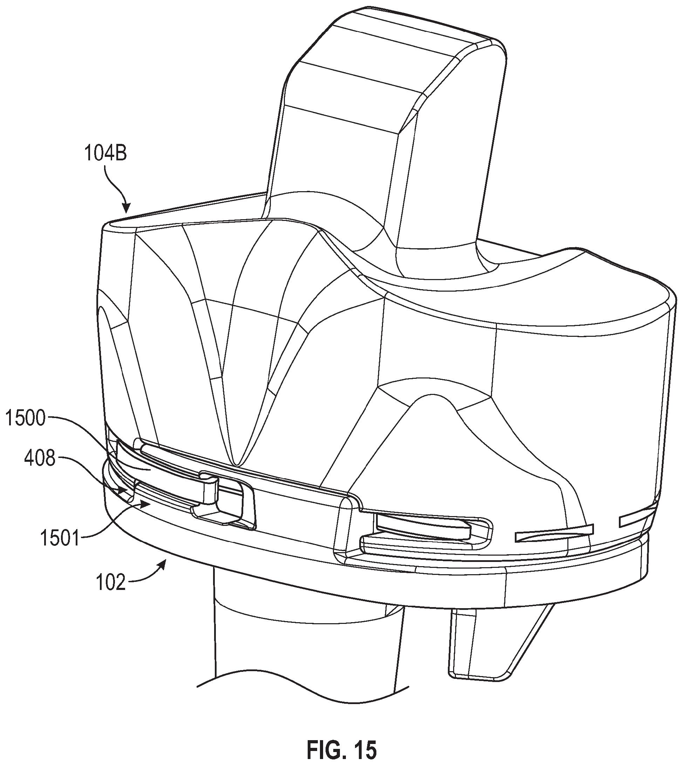

FIG. 15 shows a perspective view of a locking arrangement 1501 of the locking bar 1500 used to couple the fixed bearing 104B with the tibial prosthesis 102. In particular, the locking bar 1500 is fully inserted and coupled to the engaged with the anterior engagement feature 408 and the fixed bearing 104B.

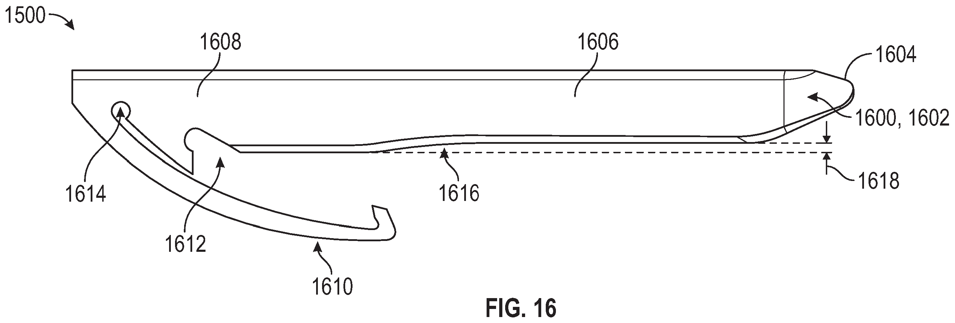

FIG. 16 shows the locking bar 1500 according to one example. The locking bar 1500 has an elongate length formed by a nose portion 1600, a intermediate portion 1606 and a main body portion 1608. The locking bar 1500 can include the nose portion 1600 including a chamfer 1602 leading to a tip 1604. The locking bar 1500 can include the intermediate portion 1606 that is connected to the nose portion 1600. The intermediate portion 1606 can be connected to the main body portion 1608. The main body portion 1608 can form an opposing end form the nose portion 1600 along the elongate length of the locking bar 1500.

The locking bar 1500 can include a deflection arm 1610 extending from the main body portion 1608. The deflection arm 1610 can be separated from the main body portion 1608 by a stop relief 1612 and a deflection arm relief 1614. A step 1616 can be provided at the junction or transition between the intermediate portion 1606 and the main body portion 1608. The step 1616 can cause a width of the locking bar 1500 to change along the elongate length thereof. The intermediate portion 1606 can have a first width and the main body portion 1608 can have a second width that is larger than the first width. The step 1616 and the resulting change in width results in a clearance 1618 (a relief) as shown in FIG. 16.

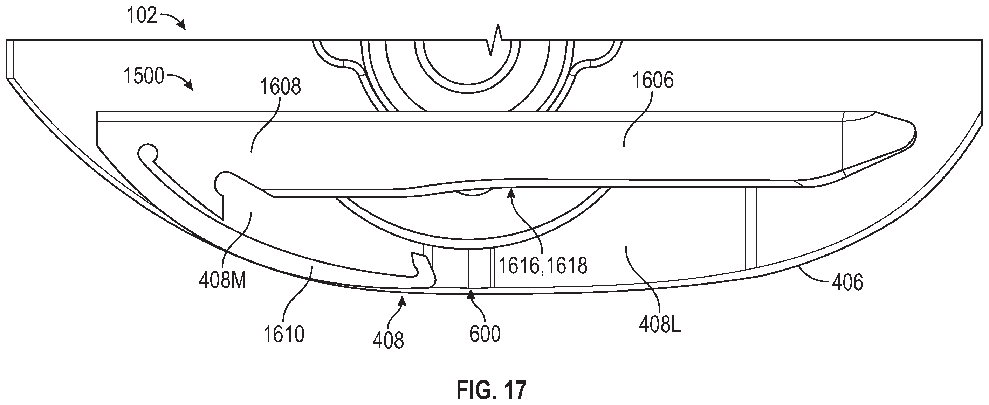

FIG. 17 shows the locking bar 1500 fully inserted and engaged with the anterior engagement feature 408. FIG. 17 does not illustrate the fixed bearing that was previously shown in FIGS. 14 and 15. FIG. 17 shows the intermediate portion 1606 engaged with the lateral portion 408L and the main body portion 1608 engaged with the medial portion 408M. The deflection arm 1610 extends around a portion (less than, up to or exceeding a majority) of the medial portion 408M such as along and generally approximating the periphery 406. The end of the deflection arm 1610 having a hook shape is received by the window 600 between the medial portion 408M and the lateral portion 408L. The step 1616 and the clearance 1618 provided by the asymmetric configuration of the locking bar 1500 allow for insertion past the asymmetrically shaped medial portion 408M into engagement with the lateral portion 408L. Thus, the locking bar 1500 is configured provide for multiple areas of engagement with the medial portion 408M and the lateral portion 408L when the locking bar 1500 is fully inserted in to the locked position shown in FIG. 17.

Referring now particularly to FIGS. 16 or 17 but also in some cases to FIG. 16, the step 1616 between the intermediate portion 1606 and the main body portion 1608 can be tapered so as to provide a ramp during insertion. The step 1616 provides a differing cross-sectional area for the main body portion 1608 as compared with the intermediate portion 1606, creating the clearance 1618 for insertion of the locking bar 1500 from the medial side. The locking bar is configured to engage with the anterior engagement feature 408 of the tibial prosthesis 102 in multiple areas/regions when fully inserted. When coupling the fixed bearing 104B (FIG. 14) to the tibial prosthesis 102, the locking bar 1500 can be inserted from the medial side, with the step 1616 providing clearance 1618 during initial insertion. The nose portion 1600 (FIG. 16) and the intermediate portion 1606 can pass through the asymmetrically shaped passage 1100 (FIG. 14) formed by the anterior engagement feature 408 and the fixed bearing 104B without engaging the medial portion 408M. The main body portion 1608 and intermediate portion 1608 when fully inserted can engage with the anterior engagement feature 408 by seating between the anterior engagement feature 408 and the fixed bearing 104B.

The present application contemplates a method of initial insertion of the nose portion 1600 into the asymmetrically shaped passage 1100, which can be followed by sliding the intermediate portion 1606 which has a first width, and then advancing the main body portion 1608 which has a second width greater than the first. This full insertion and engagement creates a trapezoidal contact area between the intermediate portion 1606 and the main body portion 1608 and the anterior engagement feature 408 and additionally between the locking bar 1500 on a second side the fixed bearing. The step can be tapered to form a ramp that reduces interference during initial insertion.

The locking bar 1500 can include retention features such as the deflection arm 1610 to prevent undesired medial movement and is configured to create an interference with the fixed bearing to couple it onto the tibial prosthesis when the locking bar 1500 is fully inserted into the anterior engagement feature 408 as shown in FIGS. 14 and 17.

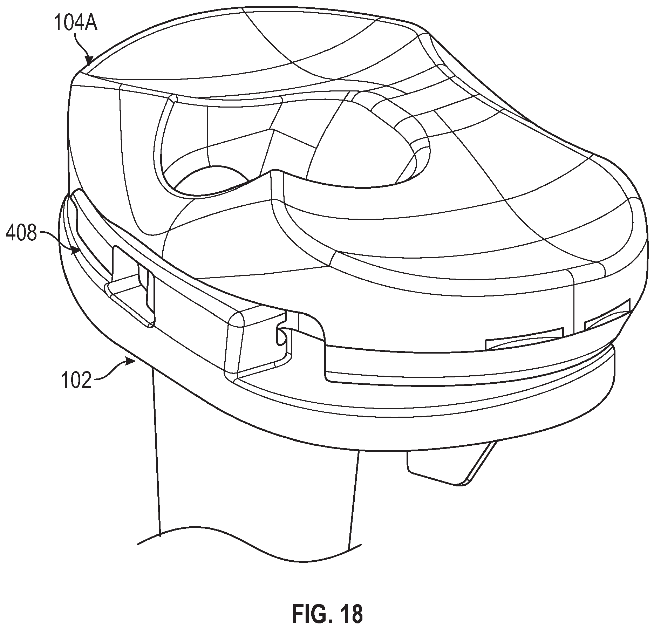

FIG. 18 is a perspective view of the mobile bearing 104A (example previously shown in FIG. 2) with the tibial prosthesis. As shown in FIG. 18, the mobile bearing 104A includes cut-outs or other features so as to accommodate the anterior engagement feature 408 of the tibial prosthesis 102.

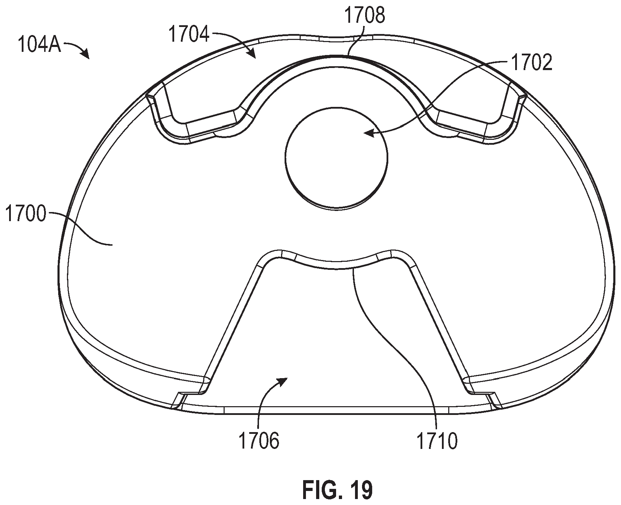

FIG. 19 shows a distal side 1700 of the mobile bearing 104A. As shown in FIG. 19, the mobile bearing 104A at the distal side 1700 can include a bore 1702, an anterior relief cut-out 1704, a posterior relief cut-out 1706, a semi-circular or arcuate projection feature 1708 and an arcuate feature 1710.

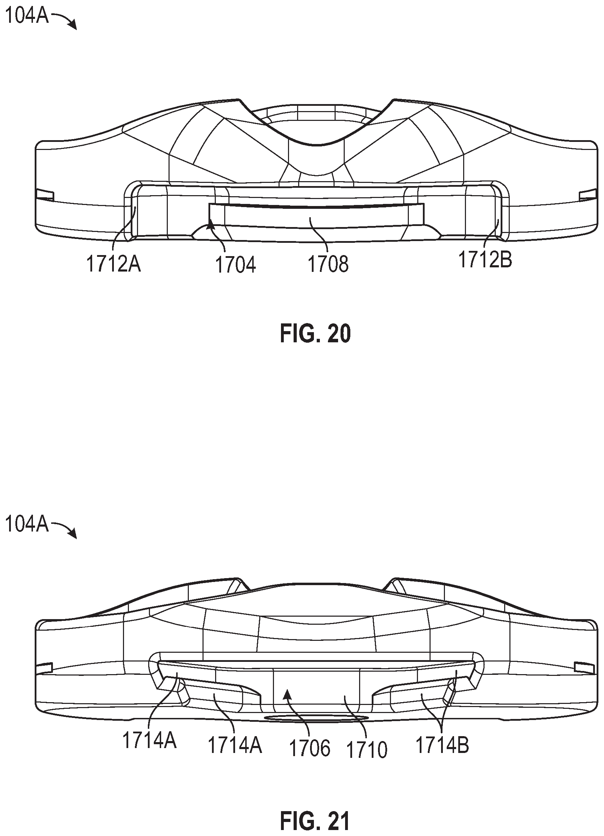

FIG. 20 shows an anterior side of the mobile bearing 104A that includes the anterior relief cut-out 1704 (a notch) and the semi-circular or arcuate projection feature 1708 within the anterior relief cut-out. FIG. 20 additionally shows a potential first stop 1712A (a surface or surfaces) and a potential second stop 1712B (surface or surfaces). The potential first stop 1712A and the potential second stop 1712B can interact with and are configured to engage the anterior engagement feature 408 (not shown in FIG. 20 but shown previously such as in FIG. 18) to halt rotation of the mobile bearing 104A according to some examples.

FIG. 21 shows a posterior side of the mobile bearing 104A that includes the posterior relief cut-out 1706 (a notch) and the arcuate feature 1710 within the posterior relief cut-out. FIG. 21 additionally shows a potential third stop 1714A (surfaces) and a potential fourth stop 1714B (surfaces). The potential third stop 1714A and the potential fourth stop 1714B can interact with and are configured to engage the posterior engagement feature 408 (not shown in FIG. 21 but shown previously in FIGS. 5, 6A and 7) to halt rotation of the mobile bearing 104A according to some examples.

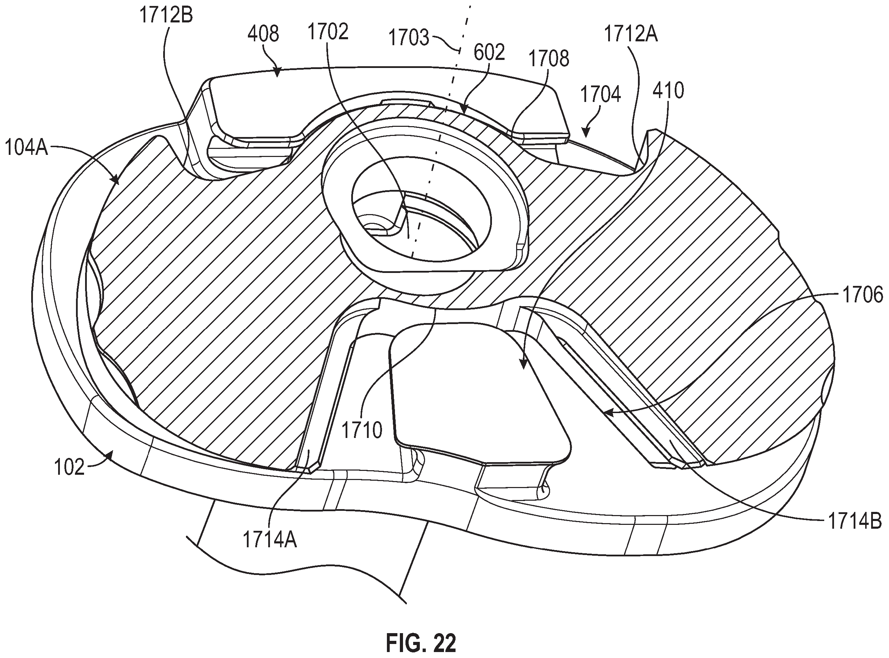

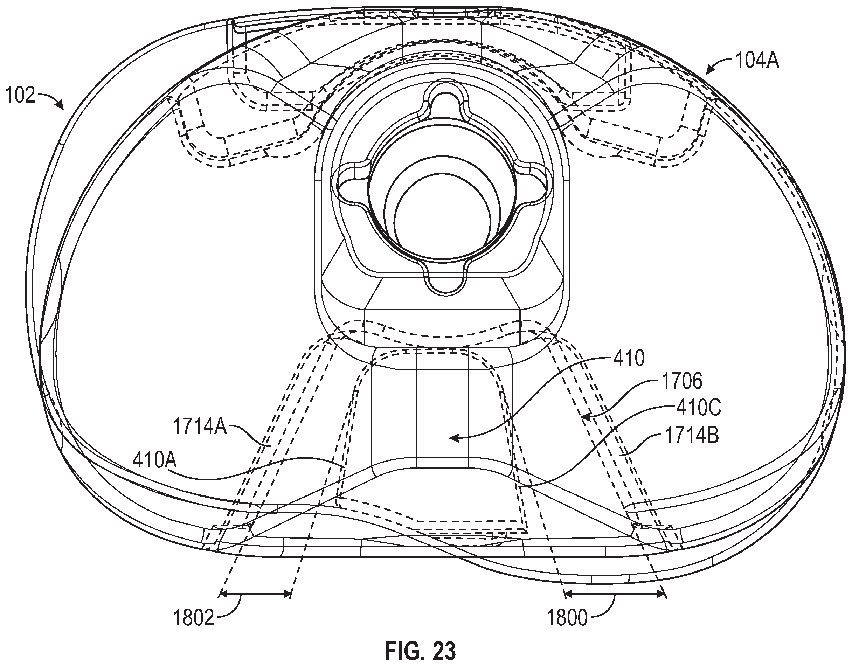

FIGS. 22 and 23 show the mobile bearing 104A arranged on the tibial prosthesis 102. FIG. 22 is a cross-section through the mobile bearing 104A and shows the bore 1702, a centerline axis 1703 of the bore 1702, the anterior relief cut-out 1704, the posterior relief cut-out 1706, the semi-circular or arcuate projection feature 1708, the arcuate feature 1710, the anterior engagement feature 408 and the posterior engagement feature 410, the potential first stop 1712A, the potential second stop 1712B, the potential third stop 1714A and the potential fourth stop 1714B. FIG. 23 shows the posterior engagement feature 410 and the posterior relief cut-out 1706 in phantom and further indicates a first distance 1800 between the first side 410A of the posterior engagement feature 410 and the potential third stop 1714A and a second distance 1802 between the third side 410C of the posterior engagement feature 410 and the potential fourth stop 1714B.

Referring now FIG. 22, the mobile bearing 104A can be configured to be coupled to the tibial baseplate 102 and to rotate about at least the centerline axis 1703 of the bore 1702, (e.g., the axis corresponding to external rotation about the axis ARA of the hinge post 208 of FIG. 2 if the hinge post 208 is utilized with the mobile bearing 104A and the tibial baseplate 102) (however note that the hinge post is optional according to some examples of the system). Thus, in some examples, the centerline axis of the bore 1703 can be co-extensive with the centerline and/or rotational axis of the hinge post 208 (see FIG. 2).

The anterior relief cut-out 1704 can be sized and shaped to provide a desired clearance between the potential first stop 1712A and the potential second stop 1712B and respective sides of the anterior engagement feature 408 when the mobile bearing 104A is in a non-fully rotated position such as shown in FIG. 22. Such desired clearance can provide for a desired rotational degree (desired degree range) of the mobile bearing 104A about the axis 1703 relative to the tibial baseplate 102. The desired degree range can be about ± 5 degrees, ± 10 degrees, ± 15 degrees, ± 20 degrees, ± 25 degrees or any desired combination of angles therebetween.

Similarly, the posterior relief cut-out 1706 can be sized and shaped to provide a desired clearance between the potential third stop 1714A and the potential fourth stop 1714B and the respective first side 410A and the third side 410C of the posterior engagement feature 410 when the mobile bearing 104A is in a non-fully rotated position such as shown in FIG. 22. Such desired clearance can provide for a desired rotational degree (desired degree range) of the mobile bearing 104A about the axis 1703 relative to the tibial baseplate 102. The desired degree range can be about ± 5 degrees, ± 10 degrees, ± 15 degrees, ± 20 degrees, ± 25 degrees or any desired combination of angles therebetween.

Thus, at least one of the potential first stop 1712A, the potential second stop 1712B, the potential third stop 1714A and the potential fourth stop 1714B can be configured to limit rotation of the mobile bearing 104A with respect to the tibial baseplate 102 about the axis 1703 in either clockwise or counterclockwise directions. Which of the potential first stop 1712A, the potential second stop 1712B, the potential third stop 1714A and the potential fourth stop 1714B limits rotation depends on the direction and amount of the rotation and a size of the mobile bearing 104A selected, for example. Additionally, the anterior engagement feature 408 can limit anterior directional movement of the mobile bearing 104A and the posterior engagement feature 410 can limit posterior directional movement of the mobile bearing 104A.

FIG. 22 additionally shows the semi-circular projection feature 1708 configured to be received in the concentric cut-out 602 of the anterior relief feature 408. Together the semi-circular projection feature 1708 and the concentric cut-out 602 interact to guide and allow for the rotation of the mobile bearing 104A about the axis 1703. The arcuate feature 1710 can have a clearance from the posterior engagement feature 410 to allow for the rotation.

Referring now to FIG. 23, the mobile bearing 104A includes the posterior relief cut-out 1706 configured to receive the posterior engagement feature 410. As shown in the example of FIG. 23, the posterior relief cut-out can be asymmetrically positioned relative to the posterior engagement feature 410 when the mobile bearing 104A is coupled to the tibial prosthesis 102 and is orientated in a neutral (0 degree rotation) position as shown in FIG. 23. This asymmetric positioning provides for a difference between the first distance 1800 and the second distance 1802 such that the first distance 1800 is larger than the second distance 1802. A similar asymmetry is contemplated according to some examples between the anterior relief cut-out and the anterior engagement feature. As such, according to some examples, the anterior relief cut-out can be asymmetrically positioned relative to the anterior engagement feature when the mobile bearing 104A is coupled to the tibial prosthesis 102 and is orientated in a neutral (0 degree rotation) position.

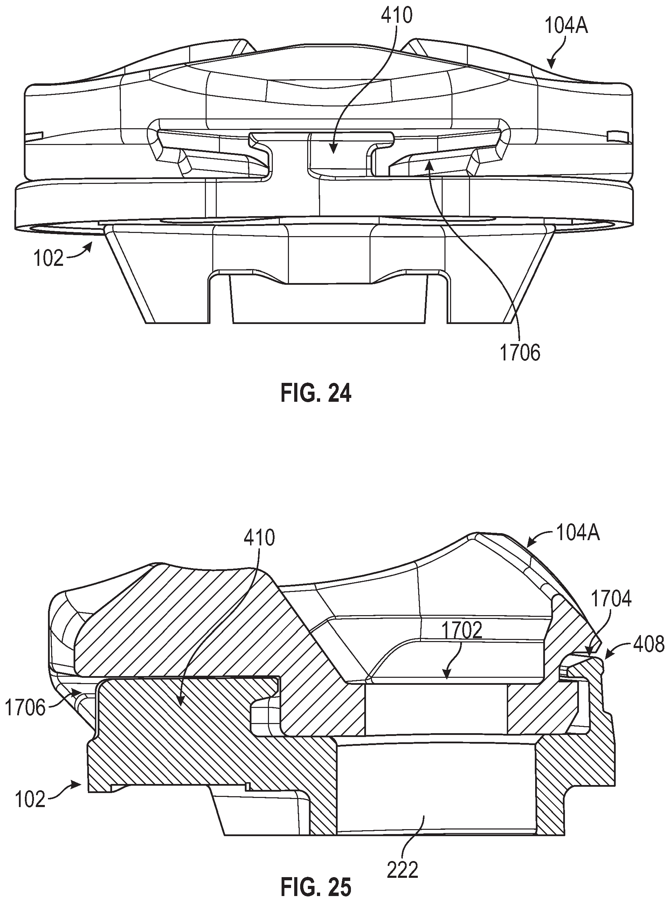

FIG. 24 shows a plan view of a posterior of the mobile bearing 104A and the tibial prosthesis 102 showing the clearance between the posterior relief cut-out 1706 and the posterior engagement feature 410 that allows for rotation of the mobile bearing 104A relative to the tibial prosthesis 102.

FIG. 25 is a cross-sectional view of the mobile bearing 104A and part of the tibial prosthesis 102. FIG. 25 shows the anterior engagement feature 408, the posterior engagement feature 410, the bore 1702, the bore 222, the anterior relief cut-out 1704 and the posterior relief cut-out 1706. As shown in FIG. 25, the anterior relief cut-out 1704 is configured to provide a clearance between the mobile bearing 104A and the anterior engagement feature 408 adjacent the anterior of the mobile bearing 104A. Similarly, the posterior relief cut-out 1706 is configured to provide a clearance between the mobile bearing 104A and the posterior engagement feature 410 adjacent a central portion of the mobile bearing 104A near but posterior of the bore 1702.

FIG. 26 is a chart 1900 showing compatibility of various different standard stock sizes components of the system discussed and illustrated previously. FIG. 26 illustrates the tibial prosthesis can be available in a family of nine different standard stock sizes, which are compatible for use with a family of seven different standard stock sizes of femoral prosthesis, eleven different standard stock sizes of fixed bearing and five standard stock sizes of locking bar.

The chart 2000 of FIG. 27 additionally shows the family of nine different standard stock sizes of tibial prosthesis, the family of seven different standard stock sizes of femoral prosthesis can be compatible for use with six different standard stock sizes of mobile bearing.

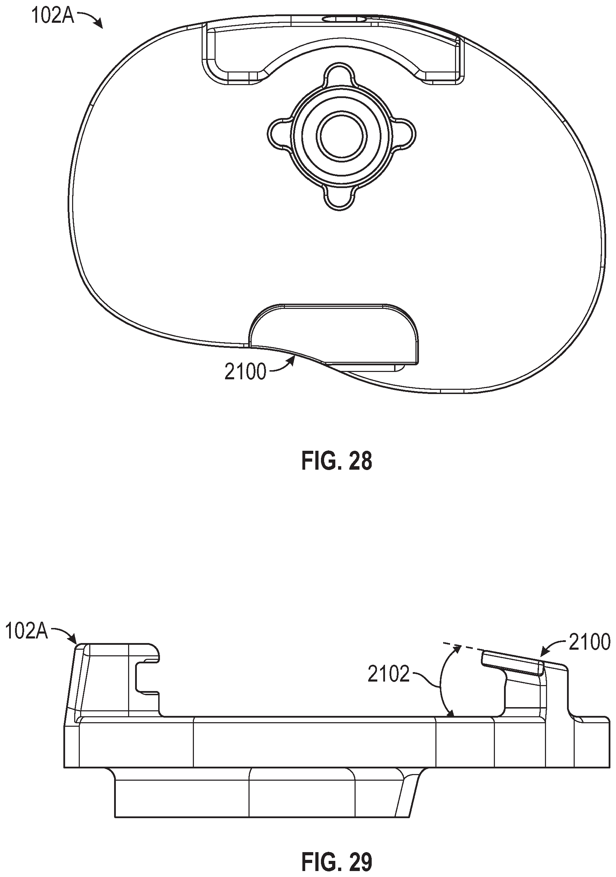

FIGS. 28 and 29 show another example of a tibial prosthesis 102A. The tibial prosthesis 102A can be configured similar to those previously discussed but can include a posterior engagement mechanism 2100 that is differently shaped and positioned relative to the posterior engagement mechanism discussed and illustrated previously. As shown in FIG. 29, the posterior engagement mechanism 2100 can be angulated anterior-posterior and proximal-distal along a proximal portion thereof so as to form an angle 2102 with a proximal surface for wedging engagement of a fixed bearing (not shown).

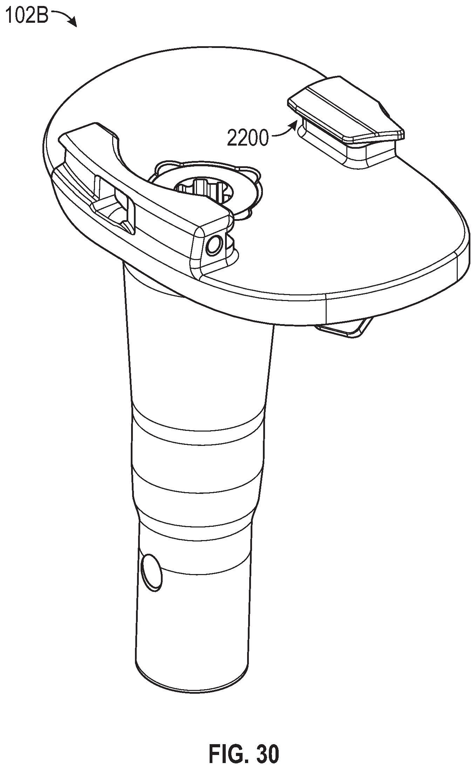

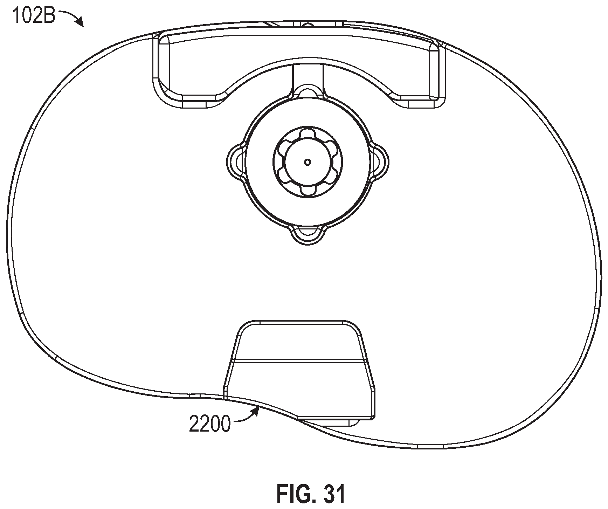

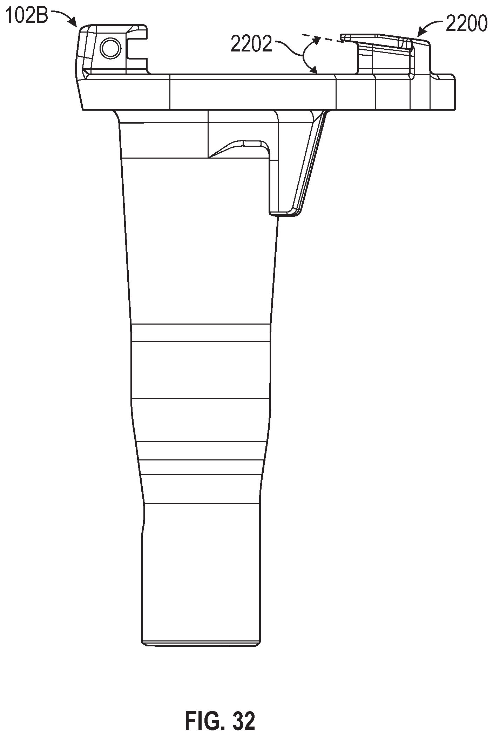

FIGS. 30-32 show another example of a tibial prosthesis 102B. The tibial prosthesis 102B can be configured similar to those previously discussed but can include a posterior engagement mechanism 2200 that is differently shaped and positioned relative to the posterior engagement mechanism discussed and illustrated previously. As shown in FIG. 32, the posterior engagement mechanism 2200 can be angulated anterior-posterior and proximal-distal along a proximal portion thereof so as to form an angle 2202 with a proximal surface for wedging engagement of a fixed bearing (not shown). The tibial prosthesis 102B differs from the tibial prosthesis 102A in that the posterior engagement mechanism 102B extends further toward the anterior and is differently shaped than the other posterior engagement mechanisms shown previously.

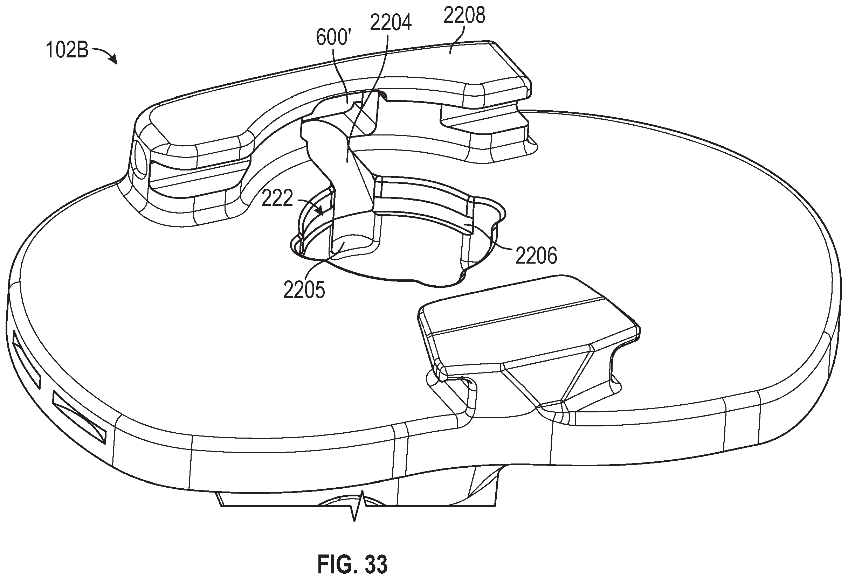

FIG. 33 is a perspective view of a proximal portion of the tibial prosthesis 102B further showing a ramp 2204, a recess 2205 and a groove 2206 in addition to the bore 222 and an anterior engagement mechanism 2208.

As shown in FIG. 33, the ramp 2204 is a sloped feature extending anterior-posterior from an anterior edge of the tibial prosthesis 102B beneath the anterior engagement mechanism 2208 to the bore 222. As shown, the ramp 2204 can be angulated to extend distally (in addition to posteriorly) to the bore 222 from the anterior edge. The ramp 2204 can be positioned below and communicating with a window 600' of the anterior engagement mechanism 2208, for example.

The recess 2205 can communicate with the ramp 2204 and can communicate with the bore 222. The recess 2205 can extend distally from the ramp 2204 into the bore 222 a distance. Recess 2205 can provide a spacing within the bore 222 from the bore plug (not shown) or other feature inserted in the bore 222.

The groove 2206 can be positioned within the bore 222 a distance below a proximal surface of the tibial prosthesis 102B. The groove 2206 can communicate with the ramp 2204 and the recess 2205. Recess 2205 can extend distal of the groove 2206.

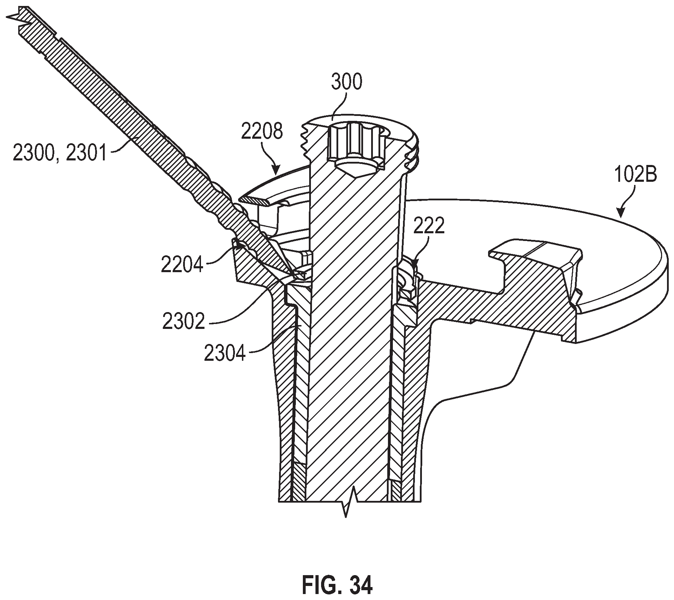

FIG. 34 is a cross-sectional view of the tibial baseplate 102B with the bore 222, the ramp 2204 and the anterior engagement mechanism 2208 and additional components including the bore plug 300, a snap ring 2302 and a tibial bushing 2304.

FIG. 34 illustrates a tool 2300, such as a trocar pin 2301, in the process of being used to release the snap ring 2302 from positioning within the bore 222. The tool 2300 is inserted into the bore 222 via the ramp 2204. The snap ring 2302 can be received in the groove 2206 (FIG. 33). A point of the trocar pin 2301 can be inserted below the snap ring 2302 via the recess 2205 (FIG. 33). Additionally, the trocar pin 2301 can be used to couple with and remove the tibial bushing 2304 from the bore 222 using the recess 2205 (FIG. 33).

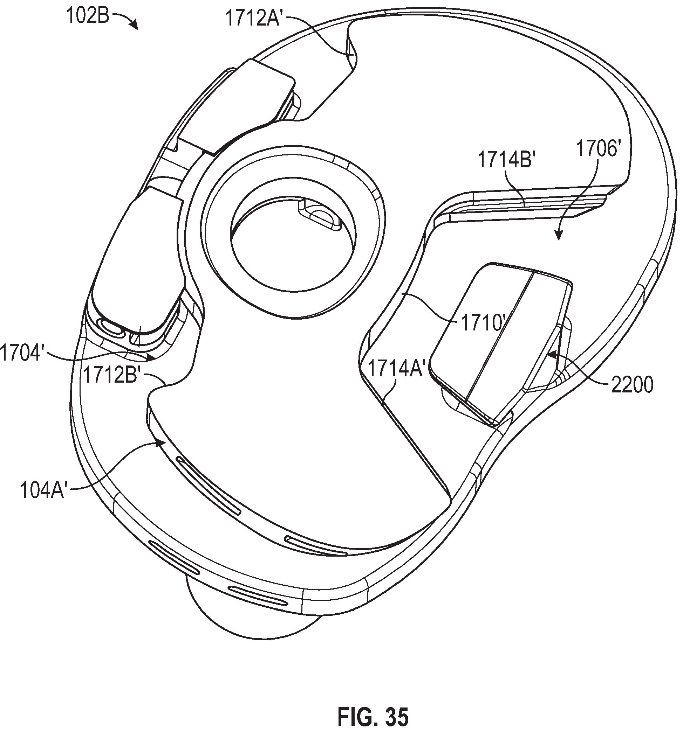

FIG. 35 is a cross-section through a mobile bearing 104A' according to another example. FIG. 35 shows an anterior relief cut-out 1704', an posterior relief cut-out 1706', an arcuate feature 1710' and the posterior engagement feature 2220 of the tibial prosthesis 102B. FIG. 35 shows a potential first stop 1712A', a potential second stop 1712B', a potential third stop 1714A' and a potential fourth stop 1714B'.

Referring now FIG. 35, the mobile bearing 104A' can be configured to be coupled to the tibial baseplate 102B and to rotate about at least a centerline axis (refer to FIG. 22) of the bore, (e.g., the axis corresponding to external rotation about the axis ARA of the hinge post 208 of FIG. 2 if the hinge post 208 is utilized with the mobile bearing 104A and the tibial baseplate 102) (however note that the hinge post is optional according to some examples of the system). Thus, in some examples, the centerline axis of the bore can be co-extensive with the centerline and/or rotational axis of the hinge post 208 (see FIG. 2).

The anterior relief cut-out 1704' can be sized and shaped to provide a desired clearance between the potential first stop 1712A' and the potential second stop 1712B' and respective sides of the anterior engagement feature when the mobile bearing 104A' is in a non-fully rotated position such as shown in FIG. 35. Such desired clearance can provide for a desired rotational degree (desired degree range) of the mobile bearing 104A' about the axis relative to the tibial baseplate 10B2. The desired degree range can be about ± 5 degrees, ± 10 degrees, ± 15 degrees, ± 20 degrees, ± 25 degrees or any desired combination of angles therebetween.

Similarly, the posterior relief cut-out 1706' can be sized and shaped to provide a desired clearance between the potential third stop 1714A' and the potential fourth stop 1714B' and the respective first side and the third side of the posterior engagement feature 2200 when the mobile bearing 104A' is in a non-fully rotated position such as shown in FIG. 35. Such desired clearance can provide for a desired rotational degree (desired degree range) of the mobile bearing 104A' about the axis relative to the tibial baseplate 102B. The desired degree range can be about ± 5 degrees, ± 10 degrees, ± 15 degrees, ± 20 degrees, ± 25 degrees or any desired combination of angles therebetween.

Thus, at least one of the potential first stop 1712A', the potential second stop 1712B', the potential third stop 1714A' and the potential fourth stop 1714B' can be configured to limit rotation of the mobile bearing 104A' with respect to the tibial baseplate 102B about the axis in either clockwise or counterclockwise directions. Which of the potential first stop 1712A', the potential second stop 1712B', the potential third stop 1714A' and the potential fourth stop 1714B' limits rotation depends on the direction and amount of the rotation and a size of the mobile bearing 104A' selected, for example. Additionally, the anterior engagement feature can limit anterior directional movement of the mobile bearing 104A' and the posterior engagement feature 2200 can limit posterior directional movement of the mobile bearing 104A'.

FIG. 35 additionally shows the semi-circular projection feature configured to be received in the concentric cut-out of the anterior relief feature. Together the semi-circular projection feature and the concentric cut-out interact to guide and allow for the rotation of the mobile bearing 104A' about the axis. The arcuate feature 1710' can have a clearance from the posterior engagement feature 2200 to allow for the rotation.

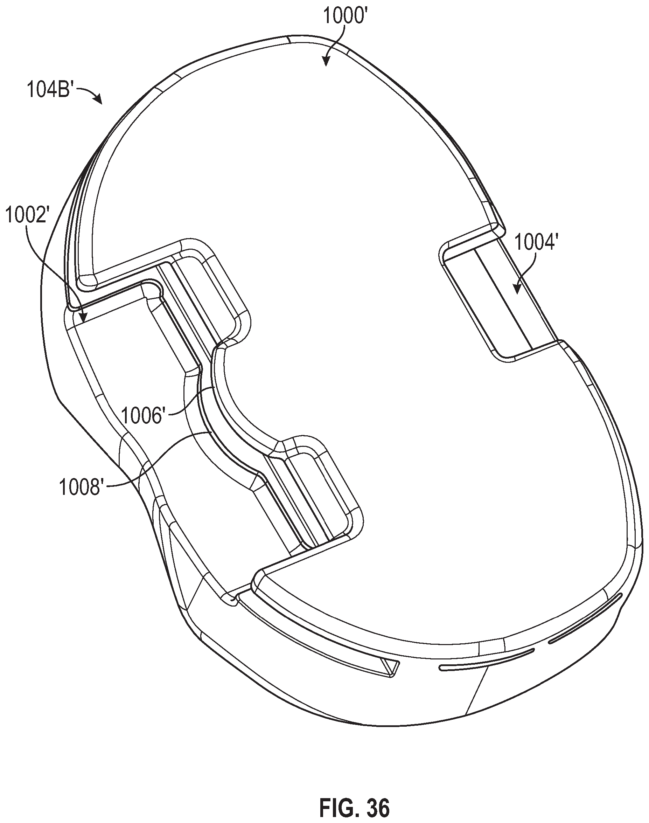

FIG. 36 illustrates a distal side 1000' of another example of a fixed bearing 104B' which can be configured for use with the tibial prosthesis of FIGS. 30-35. The fixed bearing 104B' can include one or more coupling features including an anterior coupling feature 1002' and a posterior coupling feature 1004'. The one or more coupling features including the anterior coupling feature 1002' and the posterior coupling feature 1004' can be recesses (e.g., notches, grooves, etc.) and/or projections or other features configured to couple with or otherwise interact with the anterior engagement feature and the posterior engagement feature, respectively. FIG. 36 illustrates arcuate engagement features 1006' and 1008' that are internal projections relative to other recessed features of the anterior coupling feature 1002' configured for coupling with and engaging the anterior coupling mechanism.

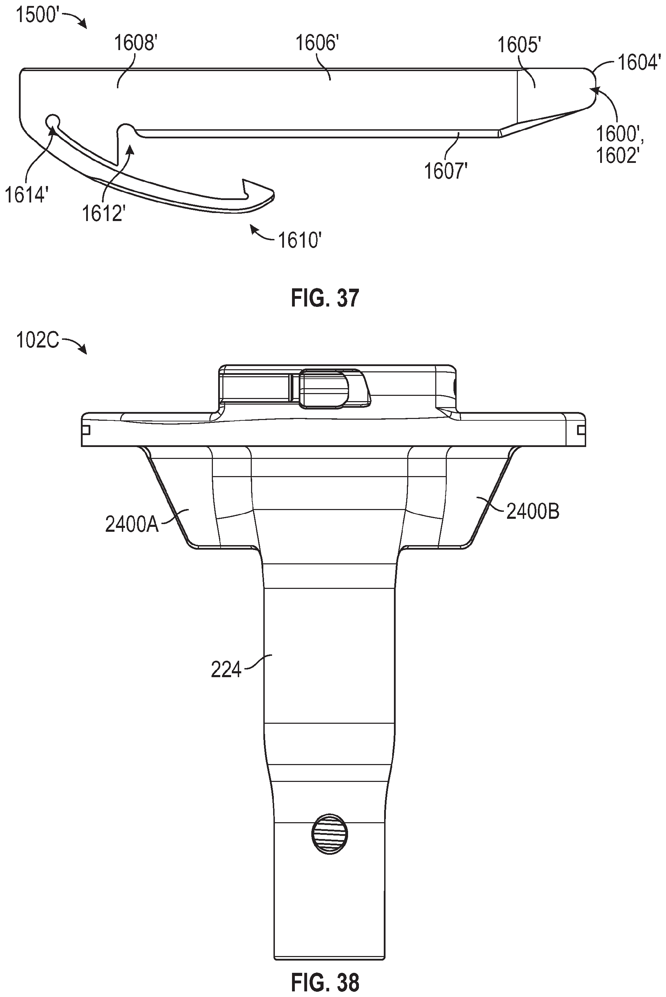

FIG. 37 shows a locking bar 1500' according to one example. The locking bar 1500' has an elongate length formed by a nose portion 1600', a intermediate portion 1606' and a main body portion 1608'. The locking bar 1500' can include the nose portion 1600' including a chamfer 1602' leading to a tip 1604'. The locking bar 1500' can include the intermediate portion 1606' that is connected to the nose portion 1600'. The intermediate portion 1606' can be connected to the main body portion 1608'. The main body portion 1608' can form an opposing end form the nose portion 1600' along the elongate length of the locking bar 1500'.

The locking bar 1500' can include a deflection arm 1610' extending from the main body portion 1608'. The deflection arm 1610' can be separated from the main body portion 1608' by a stop relief 1612' and a deflection arm relief 1614'.

The example locking bar 1500' of FIG. 37 differs from that of FIG. 16 in that the locking bar 1500' does not include a step. Rather, the locking bar 1500' relies on a relief provided by chamfer 1607' along a lateral side and a relief from an extended chamfer 1605' along and extending longitudinally back from the nose portion 1600' toward the intermediate portion 1606' for connection and mating with the bearing.
FIG. 38 is a plan view of another tibial prosthesis 102C having a modified distal shape particularly having a fully interconnected first keel 2400A and a fully interconnected second keel 2400B with the stem 224. The example of FIG. 38 has a different distal shape than the example of FIG. 3 shown previously.

### Additional Notes & Examples

The following, non-limiting examples, detail certain aspects of the present subject matter to solve the challenges and provide the benefits discussed herein, among others.

Example 1 is a knee prosthesis system optionally comprising: a tibial prosthesis configured for implantation on a tibia, the tibial prosthesis including: a distal side having a tibial keel, a proximal side opposite the distal side, a periphery extending between the proximal side and the distal side, a bore having an opening on the proximal side and extending into the tibial keel, an anterior engagement feature on the proximal side and a posterior engagement feature on the proximal side; a first bearing configured to couple with the tibial prosthesis in a fixed bearing arrangement; and a second bearing configured to couple with the tibial prosthesis in a mobile bearing arrangement where the second bearing is configured to be rotatable relative the tibial prosthesis to a desired degree range, and wherein rotation of the second bearing is limited by engagement with one of the anterior engagement feature or the posterior engagement feature; wherein the second bearing is configured to receive a hinge post therein, wherein the hinge post is configured to insert in the bore, wherein the mobile bearing arrangement with the tibial prosthesis optionally includes, a first configuration where the second bearing is rotatable relative to the tibial prosthesis about a first axis approximated by a centerline of the hinge post and a second configuration where the second bearing is rotatable relative to tibial prosthesis without the hinge post received by the second bearing and the bore.

In Example 2, the subject matter of Example 1 optionally includes, a locking bar that optionally includes a tapered step between an intermediate portion and a main body portion providing a clearance for insertion from a medial side, wherein the anterior engagement feature is configured to receive a locking bar which is configured to engage both the anterior engagement feature and the first bearing, and wherein the first bearing additionally optionally includes one or more coupling features configured to engage with a corresponding one or more coupling features of the posterior engagement feature.

In Example 3, the subject matter of Example 2 optionally includes, wherein posterior engagement feature optionally includes a wedge or truncated wedge shape with the corresponding one or more coupling features arranged on at least three sides including a generally anterior facing side, a lateral-posterior extending side and a medial-posterior extending side.

In Example 4, the subject matter of Example 3 optionally includes, wherein at least a portion of the posterior engagement feature extends to the periphery, and wherein the anterior engagement feature is positioned at or closely adjacent the periphery on an opposing side thereof from the posterior engagement feature, wherein the anterior engagement feature optionally includes a window configured to receive an end of a deflection arm of the locking bar.

In Example 5, the subject matter of Examples 1-4 optionally includes, wherein the desired degree range for the second bearing is between ± 5 to 25 degrees.

In Example 6, the subject matter of Examples 1-5 optionally includes, wherein the anterior engagement feature is asymmetrically shaped relative to an anteroposterior axis of the tibial prosthesis.

In Example 7, the subject matter of Example 6 optionally includes, wherein the anterior engagement feature optionally includes a concentric cut-out around and spaced from at least a portion the bore, wherein the concentric cut-out medializes in position concomitant with a medial shift of position of the bore when the tibial prosthesis moves from a first standard size to a second larger standard size.

In Example 8, the subject matter of Example 7 optionally includes, wherein the second bearing optionally includes a semi-circular projection feature configured to be received in the concentric cut-out, wherein together the semi-circular projection feature and the concentric cut-out provide a clearance to allow for the rotation of the second bearing.

In Example 9, the subject matter of Examples 1-8 optionally includes, wherein the first bearing optionally includes posterior gussets and a posterior bridge configured to increase rigidity and reduce liftoff of the first bearing from the tibial prosthesis.

In Example 10, the subject matter of Examples 1-9 optionally includes, a bore plug configured for insertion in the bore via the opening on the proximal side when the tibial prosthesis.

In Example 11, the subject matter of Example 10 optionally includes, a tibial bushing with anti-rotation lobes configured for insertion in the bore via the opening when the tibial prosthesis is used in the first configuration with the hinge post.

In Example 12, the subject matter of Examples 1-11 optionally includes, wherein the anterior engagement feature and the posterior engagement feature are projections from a proximal surface of the tibial prosthesis, and wherein the anterior engagement feature and the posterior engagement feature are the only projections from the proximal surface thereby allowing for the rotation of the second bearing.

In Example 13, the subject matter of Examples 1-12 optionally includes, wherein the second bearing optionally includes a posterior relief cut-out configured to receive the posterior engagement feature, wherein the posterior relief cut-out is asymmetrically positioned relative to the posterior engagement feature when the second bearing is coupled to the tibial prosthesis and orientated in a neutral rotational position.

In Example 14, the subject matter of Examples 1-13 optionally includes, a plurality of femoral prostheses configured to couple with a femur, wherein the plurality of femoral prostheses include at least three different standard femoral prosthesis families including a constrained knee prosthesis family having the hinge post, and wherein at least two of the plurality of femoral prostheses are each configured to articulate with a respective one of the first bearing or the second bearing.

In Example 15, the subject matter of Example 14 optionally includes, wherein the system optionally includes nine standard stock sizes of tibial prosthesis, seven standard stock sizes for at least one of the plurality of femoral protheses, eleven standard stock sizes of the first bearing, and six standard stock sizes of the second bearing.

In Example 16, the subject matter of Examples 1-15 optionally includes, wherein the anterior engagement feature optionally includes a medial portion and a lateral portion, wherein the lateral portion has a greater anterior-posterior thickness than the medial portion.

In Example 17, the subject matter of Example 16 optionally includes, wherein the lateral portion has a length in a medial-lateral direction that exceeds a length of the medial portion in the medial-lateral direction, wherein relative sizes of the lateral portion and the medial portion change concomitant with a medial shift of position of the bore when the tibial prosthesis moves from a first standard size to a second larger standard size.

In Example 18, the subject matter of Examples 1-17 optionally includes, wherein the first bearing and the second bearing are two of at least four bearings of different standard design families.

In Example 19, the subject matter of Examples 1-18 optionally includes, wherein the hinge post is rotatable and configured to luxate relative to the tibial prosthesis and the second bearing.

Example 20 is a method of implanting a knee prosthesis system comprising: providing a tibial prosthesis having a distal side with a tibial keel, a proximal side, a bore extending into the keel and having an opening on the proximal side, an anterior engagement feature and a posterior engagement feature; selecting between a first bearing for a fixed bearing arrangement and a second bearing for a mobile bearing arrangement; coupling one of the first bearing or the second bearing to the tibial prosthesis, wherein the second bearing is configured to be rotatable relative to the tibial prosthesis with or without a hinge post received by the second bearing and the bore of the tibial prosthesis.

In Example 21, the subject matter of Example 20 optionally includes, inserting a locking bar having a tapered step from a medial side when the first bearing is selected; engaging the locking bar with both the anterior engagement feature and the first bearing; and engaging one or more coupling features of the first bearing with corresponding coupling features of the posterior engagement feature in an interference fit.

In Example 22, the subject matter of Example 21 optionally includes, wherein engaging the coupling features optionally includes engaging surfaces on a generally anterior facing side, a lateral-posterior extending side and a medial-posterior extending side of a wedge shaped or truncated wedge shape of the posterior engagement feature.

In Example 23, the subject matter of Example 22 optionally includes, positioning the anterior engagement feature at or adjacent a periphery of the tibial prosthesis between the distal side and the proximal side; positioning the posterior engagement feature which optionally includes a portion that extends to the periphery opposite the anterior engagement feature; and receiving an end of a deflection arm of the locking bar in a window of the anterior engagement feature.

In Example 24, the subject matter of Examples 20-23 optionally includes, wherein coupling the second bearing optionally includes providing rotation of the second bearing through a range of between ± 5 to 25 degrees.

In Example 25, the subject matter of Examples 20-24 optionally includes, wherein the anterior engagement feature has an asymmetric shape relative to an anteroposterior axis of the tibial prosthesis.

In Example 26, the subject matter of Example 25 optionally includes, medializing a concentric cut-out of the anterior engagement feature around the bore when moving from a first standard size to a second larger standard size of the tibial prosthesis.

In Example 27, the subject matter of Example 26 optionally includes, wherein coupling the second bearing optionally includes receiving a semi-circular projection feature of the second bearing in the concentric cut-out to allow rotation.

In Example 28, the subject matter of Examples 20-27 optionally includes, wherein the first bearing optionally includes posterior gussets and a posterior bridge.

In Example 29, the subject matter of Examples 20-28 optionally includes, inserting a bore plug in the bore via the opening from a proximal location when using either the first bearing or the second bearing.

In Example 30, the subject matter of Example 29 optionally includes, replacing the bore plug with a tibial bushing when using the second bearing with the hinge post.

In Example 31, the subject matter of Examples 20-30 optionally includes, wherein providing the tibial prosthesis optionally includes having the anterior engagement feature and the posterior engagement feature as the only projections from a proximal surface of the proximal side to allow rotation of the second bearing.

In Example 32, the subject matter of Examples 20-31 optionally includes, wherein coupling the second bearing optionally includes asymmetrically positioning a posterior relief cut-out of the second bearing relative to the posterior engagement feature when the second bearing is in an neutral angle position relative to the tibial prosthesis.

In Example 33, the subject matter of Examples 20-32 optionally includes, providing a plurality of femoral prostheses including at least three different standard femoral prosthesis families; selecting a femoral prosthesis from the plurality of femoral prostheses; and selecting a desired bearing for use with the femoral prosthesis.

In Example 34, the subject matter of Example 33 optionally includes, providing seven different standard stock sizes of the femoral prosthesis; providing nine different standard stock sizes of the tibial prosthesis; providing eleven different standard sizes of the first bearing; and providing six different standard stock sizes of the second bearing.

In Example 35, the subject matter of Examples 20-34 optionally includes, wherein the anterior engagement feature optionally includes a medial portion and a lateral portion, wherein the lateral portion has a greater anterior-posterior thickness and a greater medial-lateral length than the medial portion.

In Example 36, the subject matter of Examples 20-35 optionally includes, providing at least four bearings of different standard design families configured to couple with the tibial prosthesis, wherein the at least four bearings include the first bearing and the second bearing.

In Example 37, the subject matter of Examples 20-36 optionally includes, wherein coupling one of the first bearing or the second bearing to the tibial prosthesis optionally includes maintaining the tibial prosthesis implanted in a tibia while selecting between the first bearing and the second bearing.

Example 38 is a tibial prosthesis comprising: a distal surface configured for implantation in a tibia; a proximal surface opposite the distal surface; a periphery extending between the proximal surface and the distal surface; a bore having an opening on the proximal surface and extending into a tibial keel; an anterior engagement feature on the proximal surface having an asymmetric shape with a medial portion configured differently from a lateral portion; and a posterior engagement feature on the proximal surface; wherein the anterior engagement feature and posterior engagement feature are configured to selectively engage with either a fixed bearing or a mobile bearing.

In Example 39, the subject matter of Example 38 optionally includes, wherein the anterior engagement feature optionally includes a concave inner profile along a side facing the bore.

In Example 40, the subject matter of Examples 38-39 optionally includes, wherein the anterior engagement feature optionally includes a window between the medial portion and the lateral portion, wherein the window is configured to receive an end of a deflection arm of a locking bar.

In Example 41, the subject matter of Examples 38-40 optionally includes, wherein the posterior engagement feature is asymmetrically arranged with respect to the mobile bearing when the mobile bearing is coupled to the tibial prosthesis and oriented in a neutral angle position relative to the tibial prosthesis.

In Example 42, the subject matter of Examples 38-41 optionally includes, wherein the lateral portion has a greater anterior-posterior thickness than the medial portion, and wherein the lateral portion has a length in a medial-lateral direction that exceeds a length of the medial portion in the medial-lateral direction.

In Example 43, the subject matter of Examples 40-42 optionally includes, wherein the anterior engagement feature optionally includes a concentric cut-out around the bore, wherein the concentric cut-out medializes in position concomitant with a medial shift of position of the bore when the tibial prosthesis moves from a first standard size to a second larger standard size.

In Example 44, the subject matter of Examples 38-43 optionally includes, wherein the posterior engagement feature optionally includes a wedge shape or a truncated wedge shape with coupling features arranged on at least: a generally anterior facing side; a lateral-posterior extending side; and a medial-posterior extending side.

In Example 45, the subject matter of Examples 38-44 optionally includes, wherein the anterior engagement feature and the posterior engagement feature are the only projections from the proximal surface.

In Example 46, the subject matter of Examples 38-45 optionally includes, wherein at least a portion of the posterior engagement feature extends to the periphery, and wherein the anterior engagement feature is positioned at or closely adjacent the periphery on an opposing side thereof from the posterior engagement feature.

In Example 47, the subject matter of Examples 38-46 optionally includes, wherein the bore is configured to selectively receive one of: a bore plug; or a tibial bushing.

Example 48 is a method of converting a knee prosthesis between configurations comprising: removing a bore plug from a bore in a tibial baseplate from a proximal location while maintaining the tibial baseplate implanted in a tibia; inserting a tibial bushing into the bore; and coupling a mobile bearing with a hinge post received by the bore to the tibial baseplate enable rotation about a centerline of the hinge post.

In Example 49, the subject matter of Example 48 optionally includes, wherein inserting the tibial bushing comprises: inserting the tibial bushing into the bore with the tibial bushing having anti-rotation lobes to prevent rotation thereof relative to the tibial prosthesis, wherein the tibial bushing is configured to prevent metal-on-metal contact between the hinge post and the tibial baseplate.

In Example 50, the subject matter of Examples 48-49 optionally includes, sequent to removing the bore plug, decoupling a fixed bearing from the tibial baseplate while maintaining the tibial baseplate implanted in the tibia.

Example 51 is a method of converting a knee prosthesis between configurations comprising: removing a tibial bushing from a bore in a tibial baseplate from a proximal location while maintaining the tibial baseplate implanted in a tibia; inserting a bore plug into the bore; and coupling a fixed bearing or mobile to the tibial baseplate.

In Example 52, the subject matter of Example 51 optionally includes, wherein the tibial bushing optionally includes anti-rotation lobes, and wherein removing the tibial bushing comprises removing the tibial bushing with the anti-rotation lobes from the bore to enable conversion from a hinged mobile bearing configuration to a fixed bearing configuration.

In Example 53, the subject matter of Examples 51-52 optionally includes, prior to removing the tibial bushing, decoupling a mobile bearing and a hinge post from the tibial baseplate while maintaining the tibial baseplate implanted in the tibia.

Example 54 is a method of preparing a knee prosthesis for implantation comprising: selecting between a mobile bearing configuration and a fixed bearing configuration; when selecting the mobile bearing configuration: inserting a tibial bushing into a bore of a tibial baseplate; and when selecting the fixed bearing configuration: inserting a bore plug into the bore of the tibial baseplate.

In Example 55, the subject matter of Example 54 optionally includes, wherein the tibial bushing is configured to receive a hinge post that couples with a mobile bearing to enable rotation of the mobile bearing about a centerline of the hinge post.

In Example 56, the subject matter of Examples 54-55 optionally includes, wherein the bore plug and the tibial bushing are each configured to be selectively inserted into and removed from the bore while maintaining the tibial baseplate implanted in a tibia to enable conversion between the mobile bearing configuration and fixed bearing configuration.

Example 57 is a locking bar for coupling a fixed bearing to a tibial prosthesis in a knee prosthesis system, the locking bar comprising: a nose portion; an intermediate portion extending from the nose portion; a main body portion extending from the intermediate portion; a deflection arm projecting from the main body portion; and a step between the intermediate portion and the main body portion providing a differing cross-sectional area for the main body portion as compared with the intermediate portion and nose portion, wherein the step creates clearance for insertion of the locking bar.

In Example 58, the subject matter of Example 57 optionally includes, wherein the step is a tapered transition that provides a ramp for insertion from a medial side of the tibial prosthesis.

In Example 59, the subject matter of Examples 57-58 optionally includes, wherein the nose portion optionally includes a chamfered surface configured to aid insertion of the locking bar, the intermediate portion has a first width and the main body portion has a second width greater than the first width.

In Example 60, the subject matter of Examples 57-59 optionally includes, wherein the deflection arm is configured to extend around part of an anterior engagement feature of the tibial prosthesis to be received in a window of the anterior engagement feature.

In Example 61, the subject matter of Examples 57-60 optionally includes, wherein: the nose portion is configured to pass the anterior engagement feature without engagement during insertion of the locking bar between the tibial prosthesis and the fixed bearing; the intermediate portion is configured to pass a medial portion of the anterior engagement feature without engagement during the insertion of the locking bar but engage a lateral portion of the anterior engagement feature when the locking bar is fully inserted between the tibial prosthesis and the fixed bearing; and the main body portion is configured to engage the medial portion of the anterior engagement feature when the locking bar is fully inserted between the tibial prosthesis and the fixed bearing.

In Example 62, the subject matter of Examples 57-61 optionally includes, wherein the step is configured to provide a relief to reduce undesired interference between the locking bar, the fixed bearing and the tibial prosthesis during insertion.

In Example 63, the subject matter of Examples 57-62 optionally includes, wherein the intermediate portion and the main body portion are configured to provide a trapezoidal contact arrangement when engaged with the fixed bearing.

In Example 64, the subject matter of Examples 57-63 optionally includes, one or more retention features including the deflection arm configured to prevent an undesired medial movement of the locking bar.

In Example 65, the subject matter of Examples 57-64 optionally includes, wherein the locking bar is configured to force an interference that couples the fixed bearing with the tibial prosthesis when the locking bar is fully inserted between the fixed bearing and the tibial prosthesis.

Example 66 is a method of coupling a fixed bearing to a tibial baseplate comprising: initially inserting a locking bar from a medial side of the tibial baseplate, wherein the locking bar optionally includes, a step along an elongate length thereof between an intermediate portion and a main body portion, the step providing clearance for the intermediate portion from a medial portion of an anterior engagement feature during the initially inserting; passing at least a nose portion and the intermediate portion of the locking bar through a passage formed by the medial portion of the anterior engagement feature of the tibial baseplate and the fixed bearing; fulling inserting the locking bar to engage the main body portion with the medial portion and the intermediate portion of the locking bar with a lateral portion of the anterior locking mechanism such that the locking bar is positioned between the anterior engagement feature and the fixed bearing to couple the fixed bearing to the tibial baseplate.

In Example 67, the subject matter of Example 66 optionally includes, wherein initially inserting the locking bar optionally includes: sliding the intermediate portion having a first width; and advancing the main body portion having a second width greater than the first width.

In Example 68, the subject matter of Examples 66-67 optionally includes, receiving an end of a deflection arm of the locking bar in a window of the anterior locking mechanism.

In Example 69, the subject matter of Examples 67-68 optionally includes, wherein the step is tapered to form a ramp that reduces interference during the initially inserting of the locking bar.

In Example 70, the subject matter of Examples 66-69 optionally includes, wherein fully inserting optionally includes establishing a trapezoidal contact arrangement between the intermediate portion, the main body portion and the fixed bearing.

Example 71 is an apparatus comprising means to implement of any of Examples 1-70.

Example 72 is a system to implement of any of Examples 1-70.

Example 73 is a method to implement of any of Examples 1-70.

Example 74 is a system, method, or apparatus that includes any element or any combination of elements of any of examples 1-70.

The above-detailed description includes references to the accompanying drawings, which form a part of the detailed description. The drawings show, by way of illustration, specific embodiments that may be practiced. These embodiments are also referred to herein as "examples." Such examples may include elements in addition to those shown or described. However, the present inventors also contemplate examples in which only those elements shown or described are provided. Moreover, the present inventors also contemplate examples using any combination or permutation of those elements shown or described (or one or more aspects thereof), either with respect to a particular example (or one or more aspects thereof), or with respect to other examples (or one or more aspects thereof) shown or described herein.

All publications, patents, and patent documents referred to in this document are incorporated by reference herein in their entirety, as though individually incorporated by reference. In the event of inconsistent usages between this document and those documents so incorporated by reference, the usage in the incorporated reference(s) should be considered supplementary to that of this document; for irreconcilable inconsistencies, the usage in this document controls.

In this document, the terms "a" or "an" are used, as is common in patent documents, to include one or more than one, independent of any other instances or usages of "at least one" or "one or more." In this document, the term "or" is used to refer to a nonexclusive or, such that "A or B" includes "A but not B," "B but not A," and "A and B," unless otherwise indicated. In the appended claims, the terms "including" and "in which" are used as the plain-English equivalents of the respective terms "comprising" and "wherein." Also, in the following claims, the terms "including" and "comprising" are open-ended, that is, a system, device, article, or process that includes elements in addition to those listed after such a term in a claim are still deemed to fall within the scope of that claim. Moreover, in the following claims, the terms "first," "second," and "third," etc. are used merely as labels, and are not intended to impose numerical requirements on their objects.

The term "about," as used herein, means approximately, in the region of, roughly, or around. When the term "about" is used in conjunction with a numerical range, it modifies that range by extending the boundaries above and below the numerical values set forth. In general, the term "about" is used herein to modify a numerical value above and below the stated value by a variance of 10%. In one aspect, the term "about" means plus or minus 10% of the numerical value of the number with which it is being used. Therefore, about 50% means in the range of 45%-55%. Numerical ranges recited herein by endpoints include all numbers and fractions subsumed within that range, e.g., 1 to 5 includes 1, 1.5, 2, 2.75, 3, 3.90, 4, 4.24, and 5. Similarly, numerical ranges recited herein by endpoints include subranges subsumed within that range, e.g., 1 to 5 includes 1-1.5, 1.5-2, 2-2.75, 2.75-3, 3-3.90, 3.90-4, 4-4.24, 4.24-5, 2-5, 3-5, 1-4, and 2-4. It is also to be understood that all numbers and fractions thereof are presumed to be modified by the term "about."

The above description is intended to be illustrative, and not restrictive. For example, the above-described examples (or one or more aspects thereof) may be used in combination with each other. Other embodiments may be used, such as by one of ordinary skill in the art upon reviewing the above description. The Abstract is to allow the reader to quickly ascertain the nature of the technical disclosure and is submitted with the understanding that it will not be used to interpret or limit the scope or meaning of the claims. Also, in the above Detailed Description, various features may be grouped together to streamline the disclosure. This should not be interpreted as intending that an unclaimed disclosed feature is essential to any claim. Rather, inventive subject matter may lie in less than all features of a particular disclosed embodiment. Thus, the following claims are hereby incorporated into the Detailed Description, with each claim standing on its own as a separate embodiment. The scope of the embodiments should be determined with reference to the appended claims, along with the full scope of equivalents to which such claims are entitled.

## Claims

1. A tibial prosthesis comprising:
a distal surface configured for implantation in a tibia;
a proximal surface opposite the distal surface;
a periphery extending between the proximal surface and the distal surface;
a bore having an opening on the proximal surface and extending into a tibial keel;
an anterior engagement feature on the proximal surface having an asymmetric shape with a medial portion configured differently from a lateral portion; and
a posterior engagement feature on the proximal surface;
wherein the anterior engagement feature and posterior engagement feature are configured to selectively engage with either a fixed bearing or a mobile bearing.

2. The tibial prosthesis of claim 1, wherein the anterior engagement feature includes a concave inner profile along a side facing the bore.

3. The tibial prosthesis of any one of claims 1-2, wherein the anterior engagement feature includes a window between the medial portion and the lateral portion, wherein the window is configured to receive an end of a deflection arm of a locking bar.

4. The tibial prosthesis of any one of claims 1-3, wherein the posterior engagement feature is asymmetrically arranged with respect to the mobile bearing when the mobile bearing is coupled to the tibial prosthesis and oriented in a neutral angle position relative to the tibial prosthesis.

5. The tibial prosthesis of any one of claims 1-4, wherein the lateral portion has a greater anterior-posterior thickness than the medial portion, and wherein the lateral portion has a length in a medial-lateral direction that exceeds a length of the medial portion in the medial-lateral direction.

6. The tibial prosthesis of any one of claims 3-5, wherein the anterior engagement feature includes a concentric cut-out around the bore, wherein the concentric cut-out medializes in position concomitant with a medial shift of position of the bore when the tibial prosthesis moves from a first standard size to a second larger standard size.

7. The tibial prosthesis of any one of claims 1-6, wherein the posterior engagement feature includes a wedge shape or a truncated wedge shape with coupling features arranged on at least:
a generally anterior facing side;
a lateral-posterior extending side; and
a medial-posterior extending side.

8. The tibial prosthesis of any one of claims 1-7, wherein the anterior engagement feature and the posterior engagement feature are the only projections from the proximal surface.

9. The tibial prosthesis of any one of claims 1-8, wherein at least a portion of the posterior engagement feature extends to the periphery, and wherein the anterior engagement feature is positioned at or closely adjacent the periphery on an opposing side thereof from the posterior engagement feature.

10. The tibial prosthesis of any one of claims 1-9, wherein the bore is configured to selectively receive one of:
a bore plug; or
a tibial bushing.

11. A method of converting a knee prosthesis between configurations comprising:
removing a bore plug from a bore in a tibial baseplate from a proximal location while maintaining the tibial baseplate implanted in a tibia;
inserting a tibial bushing into the bore; and
coupling a mobile bearing with a hinge post received by the bore to the tibial baseplate enable rotation about a centerline of the hinge post.

12. The method of claim 11, wherein inserting the tibial bushing comprises:
inserting the tibial bushing into the bore with the tibial bushing having anti-rotation lobes to prevent rotation thereof relative to the tibial prosthesis, wherein the tibial bushing is configured to prevent metal-on-metal contact between the hinge post and the tibial baseplate.

13. The method of any one of claims 11-12, further comprising:
sequent to removing the bore plug, decoupling a fixed bearing from the tibial baseplate while maintaining the tibial baseplate implanted in the tibia.

14. A method of converting a knee prosthesis between configurations comprising:
removing a tibial bushing from a bore in a tibial baseplate from a proximal location while maintaining the tibial baseplate implanted in a tibia;
inserting a bore plug into the bore; and
coupling a fixed bearing or mobile to the tibial baseplate.

15. The method of claim 14, wherein the tibial bushing includes anti-rotation lobes, and wherein removing the tibial bushing comprises removing the tibial bushing with the anti-rotation lobes from the bore to enable conversion from a hinged mobile bearing configuration to a fixed bearing configuration; and
prior to removing the tibial bushing, decoupling a mobile bearing and a hinge post from the tibial baseplate while maintaining the tibial baseplate implanted in the tibia.
